# EUROPÄISCHE PATENTSCHRIFT

(11) **EP 1 102 770 B1**
(45) Veröffentlichungstag und Bekanntmachung des Hinweises auf die Patenterteilung: **20.08.2003**
(21) Anmeldenummer: 99936616.4
(22) Anmeldetag: 15.07.1999
(51) Int. Cl.: C07D 487/22, A61K 49/00

(54) **PARAMAGNETISCHE 3-,8-SUBSTITUIERTE DEUTEROPORPHYRINDERIVATE, DIESE ENTHALTENDE PHARMAZEUTISCHE MITTEL, VERFAHREN ZU IHRER HERSTELLUNG UND IHRE VERWENDUNG FÜR DAS NEKROSE- UND INFARKT-MR-IMAGING**
PARAMAGNETIC 3-,8-SUBSTITUTED DEUTEROPORPHYRIN DERIVATIVES, PHARMACEUTICAL PREPARATIONS CONTAINING SAME, METHOD FOR PRODUCING SAME AND THEIR USE IN MAGNETIC RESONANCE IMAGING OF NECROSIS AND INFARCTION
DERIVES PARAMAGNETIQUES 3-,8-SUBSTITUES DE DEUTEROPORPHYRINE, SUBSTANCES PHARMACEUTIQUES CONTENANT LESDITS DERIVES, PROCEDE DE PREPARATION DESDITS DERIVES ET LEUR UTILISATION DANS L'IRM DE LA NECROSE ET DE L'INFARCTUS

(30) Priorität: 24.07.1998 DE 19835082
(43) Veröffentlichungstag der Anmeldung: 30.05.2001
(73) Patentinhaber: SCHERING AKTIENGESELLSCHAFT, 13342 Berlin (DE)
(72) Erfinder: PLATZEK, Johannes, D-12621 Berlin (DE); NIEDBALLA, Ulrich, D-14195 Berlin (DE); RADÜCHEL, Bernd, D-13465 Berlin (DE); WEINMANN, Hanns-Joachim, D-14129 Berlin (DE); FRENZEL, Thomas, D-12247 Berlin (DE); MISSELWITZ, Bernd, D-16548 Glienicke (DE); EBERT, Wolfgang, D-15831 Mahlow (DE)
(86) Internationale Anmeldenummer: EP9905522
(87) Internationale Veröffentlichungsnummer: WO00005235

(56) Entgegenhaltungen:
- EP-A- 0 355 041
- WO-A-95/31219
- WO-A-99/43317
- DE-A- 4 232 925
- US-A- 5 284 647

## Beschreibung

Die Erfindung betrifft den in den Patentansprüchen gekennzeichneten Gegenstand, das heißt paramagnetische 3-, 8-substituierte Deuteroporphyrinderivate, diese enthaltende pharmazeutische Mittel, Verfahren zu ihrer Herstellung und ihre Verwendung für das Nekrose- und Infarkt-MR-Imaging

Detektion, Lokalisierung und Überwachung von Nekrosen oder Infarkten ist ein wichtiger Bereich in der Medizin. So ist der Myokardinfarkt nicht ein stationärer Vorgang, sondern ein dynamischer Prozess, der sich über einen längeren Zeitraum - Wochen bis Monaten - erstreckt Der Infarkt verläuft in Phasen, die nicht scharf voneinander getrennt, sondern überlappend sind. Die erste Phase, die Entwicklung des Myokardinfarktes, umfaßt die 24 Stunden nach dem Infarkt, in denen die Zerstörung sich wie eine Welle vom Subendocard zum Myocard ausbreitet. Die zweite Phase, der bereits bestehende Infarkt, umfaßt die Stabilisierung des Bereiches, in dem Faserbildung (Fibrose) als Heilprozess erfolgt Die dritte Phase, der ausgeheilte Infarkt, beginnt, nachdem alles zerstörte Gewebe durch fibröses Narbengewebe ersetzt ist. In dieser Periode findet eine umfangreiche Restrukturierung statt

Bis heute ist kein präzises und zuverlässiges Verfahren bekannt, das die aktuelle Phase eines Myokardinfarktes am lebenden Patienten bestimmbar macht. Für die Beurteilung eines Myocardinfarktes ist es von entscheidender Bedeutung zu wissen, wie groß der Anteil des bei dem Infarkt untergegangenen (verlorenen) Gewebes ist und an welcher Stelle der Verlust erfolgte, denn von dieser Kenntnis hängt die Art der Therapie ab. Infarkte erfolgen nicht nur im Myokard, sondern auch in anderen Geweben, besonders jedoch im Hirn.

Während der Infarkt in gewissem Umfang heilbar ist, können bei einer Nekrose, dem lokal begrenzten Gewebetod, nur die schädlichen Folgen für den Restorganismus verhindert oder wenigstens gemildert werden. Nekrosen können auf vielfache Weise entstehen: durch Verletzungen, Chemikalien, Sauerstoffdefizit oder Strahlung.

Wie beim Infarkt ist die Kenntnis von Umfang und Art einer Nekrose wichtig für das weitere ärztliche Vorgehen. Schon früh erfolgten daher Versuche, Detektion und Lokalisation von Infarkten und Nekrosen durch Einsatz von Kontrastmitteln bei nichtinvasiven Verfahren wie Szintigraphie oder MRI zu verbessern. In der Literatur nehmen die Versuche, Porphyrine für das Nekroseimaging einzusetzen, einen großen Raum ein. Die erzielten Resultate ergeben jedoch ein widersprüchliches Bild.
So beschreiben Winkelman und Hayes in Nature, 200, 903 (1967), daß sich Mn-5,10,15,20-Tetrakis (4-sulfonatophenyl)-porphyrin (TPPS) selektiv im nekrotischen Teil eines Tumors anreichert. Lyon et al., Magn. Res. Med. 4, 24 (1987) dagegen beobachteten, daß sich Mn-TPPS im Körper verteilt, und zwar in Niere, Leber, Tumor und nur zu einem geringen Teil in den Muskeln. Interessant ist dabei, daß die Konzentration im Tumor erst am 4. Tag ihr Maximum erreicht und das auch nur, nachdem die Autoren die Dosis auf 0,2 mmol/kg gesteigert hatten. Die Autoren sprechen daher auch von einer offenbar nichtspezifischen Aufnahme des TPPS in den Tumor.

Bockhorst et al., wiederum berichten in Acta Neurochir. 1994 [Suppl.] 60, 347, daß MnTPPS selektiv an Tumorzellen bindet. Foster et al., J. Nucl. Med. 26, 756 (1985), ihrerseits fanden, daß sich In-111 5,10,15,20-Tetrakis (4-N-methyl-pyridinium)-porphyrin (TMPyP) nicht im nekrotischen Teil, sondern in den lebenden Randschichten anreichert.

Daraus zu folgern, daß eine Porphyrintyp - Gewebe abhängige Wechselwirkung besteht, ist nicht zwingend.

In Circulation, Vol. 90, No. 4, 1994, Part 2, Page 1468, Abstr. No. 2512, berichten Ni et al., daß sie mit einem Mn-Tetraphenyl-porphyrin (Mn-TPP) und einem Gd-Mesoporphyrin (Gd-MP) Infarktbereiche gut darstellen können.

Beide Substanzen sind Gegenstand der Anmeldung WO 95/31219.

Bei szintigraphischen Verfahren liegt die eingesetzte Dosis im Nanomolbereich. Die Verträglichkeit der Substanzen spielt daher nur eine untergeordnete Rolle. Beim MR-imaging liegt die Dosis jedoch im Milimolbereich. Hier spielt die Verträglichkeit eine ganz entscheidende Rolle.

Die für MnTPP bzw. MnTPPS bestimmten geringen akuten Verträglichkeiten (LD50) schließen ihre Verwendung am Menschen aus.

Hinzu kommt, daß Porphyrine - wie auch z.B. Gd-Mesoporphyrin - dazu neigen, sich in der Haut abzulagern, was zu einer Photosensiblisierung führt. Diese Sensibilisierung kann Tage, ja sogar Wochen andauern. Bei szintigraphischen Verfahren würde dieser Effekt infolge der geringen Dosis ohne Bedeutung sein. Gegen eine breite Anwendung szintigraphischer Verfahren spricht jedoch, daß die Auflösung einer Gamma-Kamera sehr viel geringer als die ist, die beim MR-imaging zu erreichen ist.

Für das MR-imaging des Myocardinfarktes fanden auch die Gd-Komplexe der DTPA (K. Bockhorst et al., Acta Neurochir. (1997) Suppl., 60:347-349); De Roos et al., Radiology 1989; 172:717-720) und ihres Bis(methylamids) (M. Saeed et al., Radiology, 1992; 182:675-683) Verwendung. Es zeigte sich, daß beide Kontrastmittel nur in einem engen Zeitfenster eine Differenzierung zwischen gesundem und infarziertem Gewebe ermöglichen. Vergleichbare Resultate wurden auch mit der Manganverbindung der DTPA (Immunomedics, WO 94/22490) und der DPDP (Radiology 1989; 172:59-64) erreicht.

Eine deutliche Verbesserung erzielten Weissleder et al., Radiology 1992; 182:675-683, die Antimyosin an Eisenoxide (MION) koppelten. Aufgrund seiner spezifischen Struktur ist dieses Kontrastmittel nicht für das Nekroseimaging geeignet.

Es besteht daher der dringende Bedarf, Verbindungen für das MR-Infarkt- und Nekroseimaging zu haben die:
sehr gut verträglich sind,
nicht phototoxisch sind,
chemisch stabil sind,
vollständig ausgeschieden werden,
sich in Nekrosen anreichern,
sich nicht in der Haut anreichern,
eine hohe Relaxivity besitzen,
eine hohe Wasserlöslichkeit zeigen,
ein weites Zeitfenster für die Messung liefern,
eine gute Differenzierung zwischen gesundem
und nekrotischem/infarziertem Gewebe ermöglichen.

Es wurde gefunden, daß überraschenderweise Porphyrinkomplexe bestehend aus einem Liganden der allgemeinen Formel I 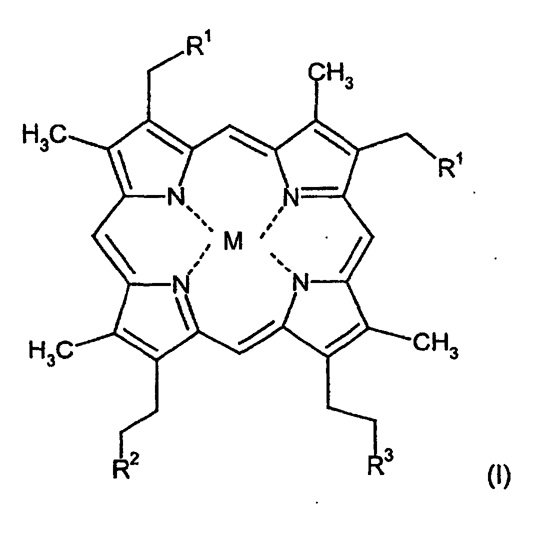 sowie mindestens einem Ion eines Elementes der Ordnungszahl 20-32, 37-39, 42-51 oder 57-83, worin
- M: für ein paramagnetisches Ion,
- R¹: für ein Wasserstoffatom, für einen geradkettigen C₁-C₆-Alkylrest, einen C₇-C₁₂-Aralkylrest oder für eine Gruppe OR' worin
R' ein Wasserstoffatom oder ein C₁-C₃-Alkylrest ist, steht,
- R² für R³,: eine Gruppe -CO-Z oder eine Gruppe -(NH)ₒ-(A)_{q}-NH-D steht, worin
Z eine Gruppe -OL ist, mit L in der Bedeutung eines anorganischen oder organischen Kations oder eines C₁-C₄-Alkylrestes ist,
A eine Phenylenoxy- oder eine durch ein oder mehrere Sauerstoffatome unterbrochene C₁-C₁₂-Alkylen- oder C₇-C₁₂ Aralkylengruppe bedeutet,
o und q unabhängig voneinander die Ziffern 0 oder 1 bedeuten und
D ein Wasserstoffatom oder eine Gruppe -CO-A-(COOL)ₒ-(H)ₘ bedeutet, mit m gleich 0 oder 1 und unter der Maßgabe, daß die Summe aus m und o gleich 1 ist,
- R³: für eine Gruppe -(C=Q)(NR⁴)ₒ-(A)_{q}-(NR⁵)-K steht,
worin Q für ein Sauerstoffatom oder für zwei Wasserstoffatome steht, R⁴ eine Gruppe -(A)_{q}-H bedeutet und
K einen Komplexbildner der allgemeinen Formel (IIa), (IIb), (IIc), (IId), (IIe) oder (IIf) bedeutet, wobei R⁵ für den Fall, daß K ein Komplexbildner der Formel (IIa) ist die gleiche Bedeutung wie R⁴ hat und R⁵ für den Fall, daß K ein Komplexbildner der Formel (IIb), (IIc), (IId) ,(IIe) oder (IIf) ist, die gleiche Bedeutung wie D hat,
mit der Maßgabe, daß eine direkte Sauerstoff-Stickstoff Bindung nicht zugelassen ist,
und K für einen Komplexbildner der allgemeinen Formel (IIa), (IIb), (IIc), (IId), (IIe) oder (IIf) 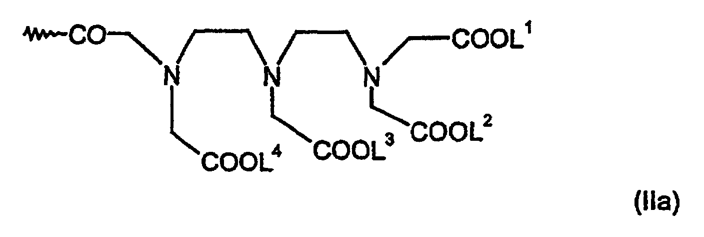 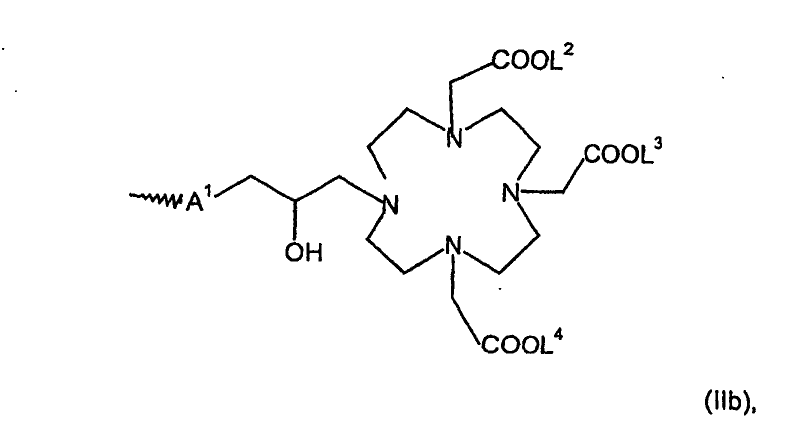 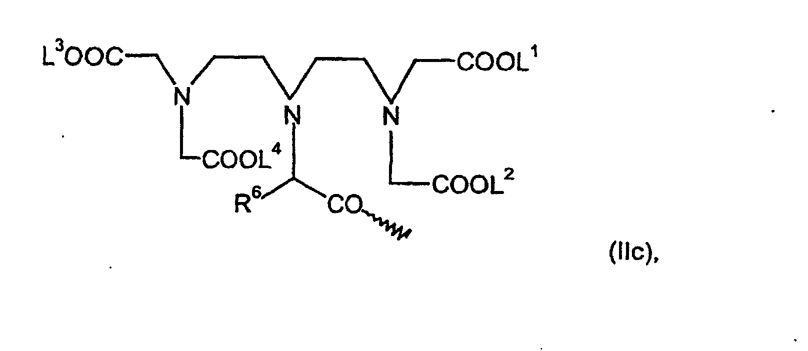 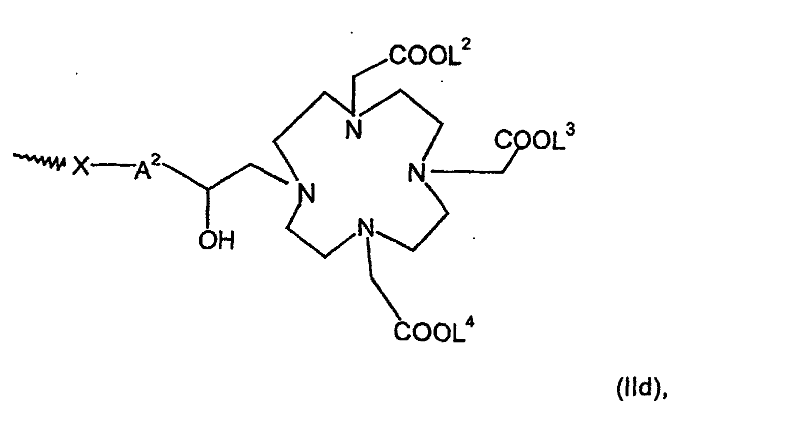 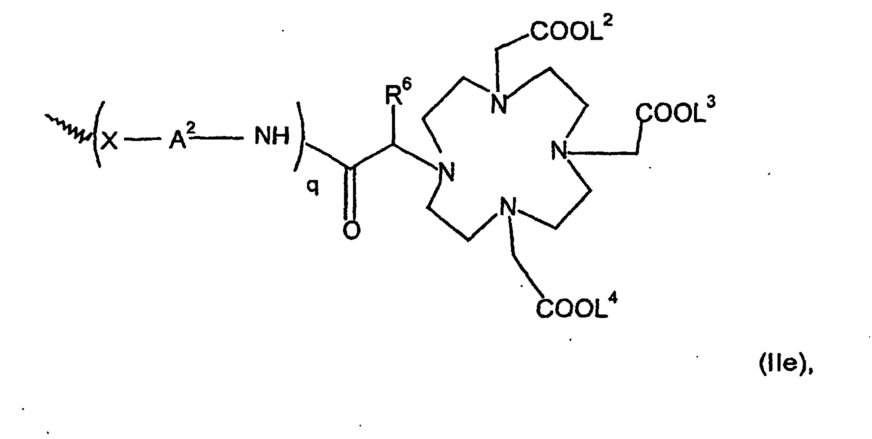 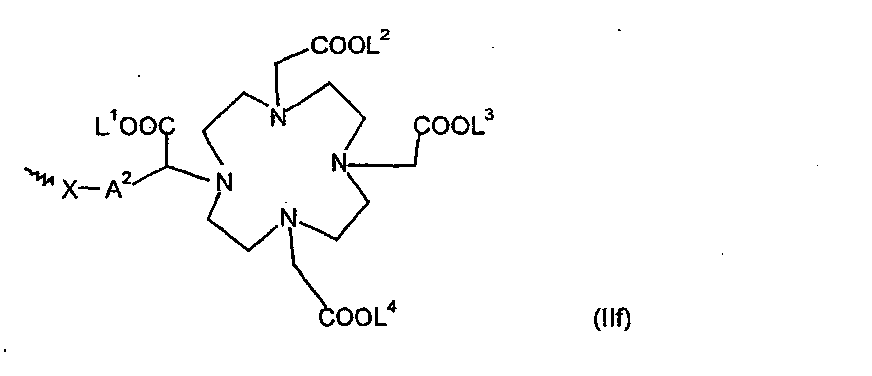
steht,
worin
q die oben angegebene Bedeutung hat,
A¹ die für A angegebene Bedeutung hat,
R⁶ für ein Wasserstoffatom, eine geradkettige oder verzweigte C₁- C₇-Alkylgruppe,
   eine Phenyl- oder Benzylgruppe,
A² für eine Phenylen-, -CH₂-NHCO-CH₂-CH (CH₂COOH) -C₆H₄-β-, -C₆H₄-O-(CH₂)₀₋₅-β, -C₆H₄-(OCH₂CH₂)₀₋₁-N(CH₂COOH)-CH₂-β oder eine gegebenenfalls durch ein oder mehrere Sauerstoffatome, 1 bis 3-NHCO-, 1 bis 3 -CONH-gruppen unterbrochene und/oder mit 1 bis 3-(CH₂)₀₋₅COOH-Gruppen substituierte C₁-C₁₂-Alkylen- oder C₇-C₁₂-Alkylengruppe, wobei β für die Bindungsstelle an X steht,
X für eine -CO- oder NHCS-gruppe und
L¹, L², L³ und L⁴ unabhängig voneinander für ein Wasserstoffatom oder ein Metallionenäquivalent eines Elements der oben genannten Ordnungszahl steht, unter den Maßgaben, daß mindestens zwei dieser Substituenten für Metallionenäquivalente stehen, und daß zum Ausgleich gegebenenfalls vorhandener Ladungen im Metalloporphyrin weitere Anionen vorhanden sind und worin freie, nicht zur Komplexierung benötigte Carbonsäuregruppen auch als Salze mit physiologisch verträglich anorganischen und/oder organischen Kationen oder als Ester oder als Amide vorliegen können,
überraschenderweise für das Nekrose- und Infarkt-MR-Imaging geeignet sind.
Sie erfüllen die an derartige Verbindungen zu stellenden Anforderungen (s.o.). Sie können auch zur Therapiekontrolle in der photodynamischen Therapie (PDT) verwendet werden.

Die erfindungsgemäßen Porphyrin-Komplexe enthalten als paramagnetisches Ion im Porphyringerüst das Eisen(III)-, Mangan(III)-, Kupfer(II)-, Cobalt(III), Chrom(III)-, Nickel(II)- oder Vanadyl(II)-Ion, wobei die drei erstgenannten bevorzugt sind.

Überraschenderweise zeigen die erfindungsgemäßen Komplexe gegenüber den bislang bekannten, strukturell ähnlichen Verbindungen eine deutlich höhere Relaxivität. Da die Relaxivität als ein Maß für die Kontrastmittelwirksamkeit einer Verbindung angesehen werden kann, gelingt bei Verwendung der erfindungsgemäßen Komplexe im Bereich der NMR-Diagnostik eine vergleichbare, positive Signalbeeinflussung schon bei einer niedrigen Dosis. Dadurch vergrößert sich der Sicherheitsabstand signifikant, für den als Richtwert das Produkt aus Relaxivität und Verträglichkeit angesehen werden kann.

Sofern eines der im Porphyrin gebundenen Ionen in einer höheren Oxidationsstufe als +2 vorliegt, so wird (werden) die überschüssige(n) Ladung(en) z.B. durch Anionen von organischen oder anorganischen Säuren, bevorzugt durch Acetat-, Chlorid-, Sulfat-, Nitrat-, Tartrat-, Succinat-, und Maleat-Ionen oder durch in R² und/oder R³ vorhandenen negativen Ladungen ausgeglichen.

Gewünschtenfalls können die Carboxylgruppen, die nicht für die Komplexierung der Metallionen benötigt werden, als Ester, als Amide oder als Salze anorganischer oder organischer Basen vorliegen. Geeignete Esterreste sind solche mit 1 bis 6 C-Atomen vorzugsweise die Ethylester, geeignete anorganische Kationen sind beispielsweise das Lithium- und das Kalium-Ion und insbesondere das Natrium-Ion. Geeignete Kationen organischer Basen sind solche von primären, sekundären oder tertiären Aminen, wie zum Beispiel Ethanolamin, Diethanolamin, Morpholin, Glucamin, N, N-Dimethylglucamin, insbesondere das Meglumin.

Bevorzugt stehen R² und R³ jeweils für die Gruppen -CONHNHK, -CONH(CH₂)₂NHK, -CONH(CH₂)₃NHK, -CONH(CH₂)₄NHK und -CONH(CH₂)₂O(CH₂)₂NHK, wobei die erste Gruppe bevorzugt ist, R² und R³ stehen bevorzugt für den gleichen Rest.
A² steht bevorzugt für eine Alkylen-, -CH₂-, -(CH₂)₂-, -CH₂OC₆H₄-β, -CH₂ OCH₂- - C₆H₄-, -CH₂-NHCO-CH₂-CH(CH₂COOH)-C₆H₄-β, -C₆H₄-OCH₂-β oder -C₆H₄-OCH₂CH₂-N(CH₂COOH)CH₂-β Gruppe, wobei β für die Bindungsstelle an X steht. X steht bevorzugt für die CO-gruppe.
R6 steht bevorzugt für ein Wasserstoffatom oder eine Methylgruppe.

Bevorzugt steht A für

-CH₂-CH₂-

-CH₂CH₂-CH₂-

-CH₂CH₂CH₂CH₂-

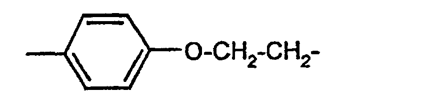

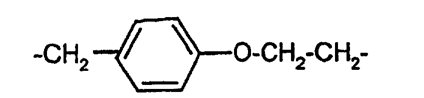

-CH₂CH₂O-CH₂CH₂-

-CH₂CH₂O-CH₂CH₂-O-CH₂CH₂-

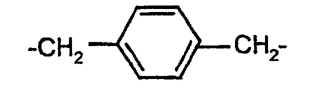

-(CH₂)₅-

-(CH₂)₆-

-(CH₂)₁₀-

-(CH₂CH₂-O)₂-CH₂CH₂-O-CH₂CH₂-

q steht bevorzugt für die Ziffer 0.

Als besondere Verbindungen seien (mu-[{16, 16'-[Chloromangan(III)-7,12-diethyl-3, 8, 13, 17-tetramethylporphyrin-2, 18-diyl]-bis[3, 6, 9-tris(carboxymethyl)-11, 14-dioxo-3, 6, 9, 12, 13-pentaazahexadecanoato]}(8-)]}-digadolinato(2-),-Dinatrium, {mu[{16, 16'-[Chloroeisen(III)-7, 12-diethyl-3, 8, 13, 17-tetramethylporphyrin-2, 18-diyl]-bis[3, 6, 9-tris(carboxymethyl)-11, 14-dioxo-3, 6, 9, 12, 13-pentaazahexadecanoato]}(8-)]}digadolinato(2-),-Dinatrium, {mu[{16, 16'-[Kupfer(II)-7, 12-diethyl-3, 8, 13, 17-tetramethylporphyrin-2, 18-diyl]-bis[3, 6, 9-tris(carboxymethyl)-11, 14-dioxo-3, 6, 9, 12, 13-pentaazahexadecanoato]}(8-)]}-digadolinato(2-),-Dinatrium genannt.

Als Komplexbildnerrest K seien vorzugsweise Derivate der Diethylentriaminpentaessigsäure und der 1,4,7,10-Tetraazacyclododecan-1,4,7-triessigsäure genannt, die über einen linker an das jeweilige Prophyrin gebunden sind.

Die Herstellung der Komplexverbindungen der allgemeinen Formel I erfolgt nach literaturbekannten Methoden ( s. z.B. DE 4232925 für II a und II b; s. z.B. DE 19507822, DE 19580858 und DE 19507819 für III c; s. z.B. US-5,053,503, WO 96/02669, WO 96/01655, EP 0430863, EP 255471, US-5,277,895, EP 0232751, US-4,885,363 für II d, II e und II f).

Die Verbindungen worin R2 und R3 für CONHNHK-gruppen stehen, sind bevorzugt. Die Synthese des hierfür als Edukt benötigten 3, 3'-(7, 12-Diethyl-3, 8, 13, 17-tetramethylporphyrin-2, 18-diyl)di(propanohydrazid) wird in Z. Physiol Chem. 241, 209 (1936) beschrieben.

Die Einführung der gewünschten Metalle (z.B. Mn) in die Porphyrine erfolgt nach literaturbekannten Methoden (z.B. The Porphyrins, ed. D. Dolphin, Academic Press, New York 1980, Vol. V, p. 459; DE 4232925), wobei im wesentlichen zu nennen sind:
a) die Substitution der pyrrolischen NH's (durch Erwärmen des metallfreien Liganden mit dem entsprechenden Metallsalz , vorzugsweise dem Acetat, gegebenenfalls unter Zusatz von säurepuffernden Mitteln, wie z.B. Natriumacetat , in einem polaren Lösungsmittel) oder
b) die "Umkomplexierung", bei der ein bereits vom Liganden komplexiertes Metall durch das gewünschte Metall verdrängt wird.
Als Lösungsmittel sind vor allem polare Solventien, wie z.B. Methanol, Eisessig, Dimethylformamid, Chloroform und Wasser geeignet.

Die Einführung des paramagnetischen Metalls M in das Porphyrinsystem kann vor oder nach Anknüpfung des Komplexbildner-Restes K erfolgen. Dadurch wird eine besonders flexible Vorgehensweise für die Synthese der erfindungsgemäßen Verbindungen ermöglicht.
Die Chelatisierung des Restes K erfolgt in literaturbekannter Weise (siehe z.B.
DE 34 01 052) indem das Metalloxid oder -salz (z.B. das Nitrat, Acetat, Carbonat, Chlorid oder Sulfat) des jeweils gewünschten Metalls, in polaren Lösungsmitteln wie Wasser oder wäßrigen Alkoholen suspendiert oder gelöst wird und mit der entsprechenden Menge des komplexbildenden Liganden umgesetzt wird. Soweit gewünscht, können vorhandene acide Wasserstoffatome oder Säuregruppen durch Kationen anorganischer und/oder organischer Basen oder Aminosäuren substituiert werden.

Die Neutralisation erfolgt dabei mit Hilfe anorganischer Basen wie z.B. Alkali- oder Erdalkali-hydroxiden, -carbonaten oder -bicarbonaten und/oder organischer Basen wie unter anderem primärer, sekundärer und tertiärer Amine, wie z.B. Ethanolamin, Morpholin, Glucamin, N-Methyl- und N,N-Dimethylglucamin, sowie basischer Aminosäuren, wie z.B. Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (z.B. Methanol, Ethanol, Isopropanol), niederen Ketonen (z.B. Aceton), polaren Ethern (z.B. Tetrahydrofuran, Dioxan, 1,2-Dimethoxyethan) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte des zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Eine andere Möglichkeit, zu neutralen Komplexverbindungen zu kommen, besteht darin, die verbleibenden Säuregruppen im Komplex ganz oder teilweise in Ester zu überführen. Dies kann durch nachträgliche Reaktion am fertigen Komplex geschehen (z.B. durch erschöpfende Umsetzung der freien Carboxy-Gruppen mit Dimethylsulfat).

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie z.B. Tromethamin), geringe Zusätze von Komplexbildnern (wie z.B. Diethylentriaminpentaessigsäure) oder, falls erforderlich, Elektrolyte wie z. B. Natriumchlorid oder, falls erforderlich, Antioxidantien wie z.B. Ascorbinsäure.

Sind für die enterale Verabreichung oder andere Zwecke Suspensionen oder Lösungen der erfindungsgemäßen Mittel in Wasser oder in physiologischer Salzlösung erwünscht, werden sie mit einem oder mehreren in der Galenik üblichen Hilfsstoff(en) (z.B. Methylcellulose, Lactose, Mannit) und/oder Tensid(en) (z.B. Lecithine, Tween®, Myrj®) und/oder Aromastoffen zur Geschmackskorrektur (z.B. etherischen Ölen) gemischt.

Prinzipiell ist es auch möglich, die erfindungsgemäßen pharmazeutischen Mittel auch ohne Isolierung der Komplexsalze herzustellen, In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 20 µmol/L bis 200 mmol/L des Komplexsalzes und werden in der Regel in Mengen von 1 µmol bis 2 mmol/kg Körpergewicht dosiert, sowohl in ihrer Anwendung für das Nekrose- und Infarkt-MR-Imaging als auch für die Therapiekontrolle mittels MRI-Diagnostik. Sie sind zur enteralen und parenteralen Applikation bestimmt oder werden mit den Methoden der interventionellen Radiologie appliziert.

Die erfindungsgemäßen Mittel erfüllen die vielfältigen Voraussetzungen für die Eignung als Mittel für MRI-Kontrastmittel. So sind sie hervorrragend dazu geeignet, nach Applikation durch Erhöhung der Signalintensität das mit Hilfe des Kernspintomographen erhaltene Bild in seiner Aussagekraft zu verbessern. Ferner zeigen sie die.hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die Verbindungen der allgemeinen Formel I sind auch zur Darstellung des Intravasalraums (blood-pool) geeignet.

Die gute Wasserlöslichkeit der erfindungsgemäßen Mittel erlaubt es hochkonzentrierte Lösungen herzustellen, damit die Volumenbelastung des Kreislaufs in vertretbaren Grenzen zu halten und die Verdünnung durch Körperflüssigkeit auszugleichen. Weiterhin weisen die erfindungsgemäßen Mittel nicht nur eine hohe Stabilität in vitro auf, sondern auch eine überraschend hohe Stabilität in vivo, so daß eine Freigabe oder ein Austausch der in den Komplexen nicht konvalent gebundenen - an sich giftigen - Ionen innerhalb der Zeit, in der die Kontrastmittel vollständig wieder ausgeschieden werden, zu vernachlässigen ist.

Die Erfindung wird durch die nachfolgenden Beispiele erläutert.

### Beispiel 1

### a) Acetato [7, 12-diethyl-3, 8, 13, 17-tetramethylporphyrin-2, 18-dipropionylhydrazinato(2-)-K N21, K N22, K N23, K N24]-Eisen

1190 mg (2 mmol) 3,3'-(7, 12- Diethyl-3, 8, 13, 17-tetramethylporphyrin-2, 18-diyl)-di(propanohydrazid), hergestellt analog zu H. Fischer, E. Haarer und F, Stadler, Z. Physiol. Chem. 241, 209 (1936), und 706,36 mg (2 mmol) Eisen(III)-acetylacetonat werden in 150 ml Essigsäure/100 ml Chloroform 5 Stunden auf 70°C erhitzt. Dann wird im Vakuum eingedampft, der Rückstand in Wasser aufgeschlämmt, abfiltriert und mit Wasser gewaschen. Das getrocknete Rohprodukt wird aus Pyridin/Diethylether umkristallisiert. Ausbeute: 1,25 g (89 % d. Th.) rotbraunes Pulver

| Elementaranalyse: | | | | |
|---|---|---|---|---|
| ber. | C 61,10 | H 6,12 | N 15,84 | Fe 7,89 |
| gef. | C 60,95 | H 6,31 | N 15,70 | Fe 7,68 |

### b) Acetato [7, 12-diethyl-3, 8, 13, 17-tetramethyl-2, 18-bis {3, 6, 16-trioxo-8, 11, 14-tris(carboxymethyl) 17-oxa-4, 5, 8, 11, 14-pentaazanonadec-1-yl}porphyinato (3-)]-Eisen

806,8 mg (2 mmol) 3-Ethoxy-carbonylmethyl-6-[2-(2,6-dioxomorpholino)ethyl]-3,6-diazaoctandi-säure (DTPA-monoethylester-monoanhydrid) werden in 250 ml absolutem Dimethylformamid suspendiert. Man überschichtet mit Stickstoff, setzt 1,0 g (10 mmol) Triethylamin und 707 mg (1 mmol) der Titelverbindung aus Beispiel 1a zu und rührt das resultierende Reaktionsgemisch 3 Tage bei Raumtemperatur. Nach beendeter Reaktion wird filtriert, das Lösungsmittel im Vakuum abgezogen und das verbleibende Öl mit 500 ml Diethylether verrieben. Der ausgefallene Feststoff wird abfiltriert und mit Diethylether und n-Hexan gewaschen. Zur Reinigung wird an Kieselgel RP-18 chromatographiert (Eluens: H₂O/Tetrahydrofuran: 0-30 %).
Ausbeute: 1,62 g (93 % d. Th.) rotbraunes Pulver
Wassergehalt: 5,2 %

| Elementaranalyse (berechnet auf wasserfreie Substanz): | | | | |
|---|---|---|---|---|
| ber. | C 53,93 | H 6,19 | N 12,95 | Fe 3,69 |
| gef. | C 53,75 | H 6,37 | N 12,81 | Fe 3,49 |

### c) Chloro [7, 12-diethyl-3, 8, 13, 17-tetramethyl-2, 18-bis{3, 6, 18-trioxo-8, 11, 14-tris(carboxymethyl)-4, 5, 8, 11, 14-pentaazahexadecanato}porphinato(3-)]-Eisen

1,59 g (0,660 mmol) des unter Beispiel 1b hergestellten Liganden werden in 400 ml Wasser gelöst. Durch Zugabe von 10 molarer wäßriger Natronlauge wird pH 13 eingestellt und 5 Stunden bei Raumtemperatur gerührt. Nach vollzogener Verseifung der Estergruppen wird mit konzentrierter Salzsäure pH 3 eingestellt. Man dampft im Vakuum zur Trockne ein. Der Rückstand wird an Kieselgel RP 18 chromatographiert (Eluens: H₂O/Tetrahydrofuran/Gradient).
Ausbeute: 0,89 g (95 % d. Th.) rotbraunes Pulver
Wassergehalt: 6,1 %

| Elementaranalyse (berechnet auf wasserfreie Substanz): | | | | | |
|---|---|---|---|---|---|
| ber. | C 51,90 | H 5,76 | N 13,67 | Fe 3,89 | Cl 2,47 |
| gef. | C 51,75 | H 5,88 | N 13,54 | Fe 3,75 | Cl 2,38 |

### d) {mu-[{16,16'-[Chloroeisen(III)-7, 12-diethyl-3, 8, 13, 17-tetramethylporphyrin-2, 18-diyl]-bis[3, 6, 9-tris(carboxymethyl)-11,14-dioxo-3, 6, 9, 12, 13-pentaazahexadecanoato]}(8-)]}-digadolinato(2-),

0,86 g (0,599 mmol)) des unter Beispiel 1c hergestellten Liganden werden in 400 ml Wasser gelöst und abwechselnd portionsweise 316,3 mg 1,2 mmol) Gadoliniumchlorid und 2n wäßrige Natronlauge zugesetzt, so daß der pH-Wert des Reaktionsgemisches stets zwischen 6,8 und 7,2 pendelt. Ist alles Gadoliniumchlorid zugesetzt, wird über Nacht bei Raumtemperatur nachgerührt. Zur Aufarbeitung wird das Lösungsmittel im Vakuum abgezogen und der Rückstand an Kieselgel RP-18 chromatographiert (Eluens: H₂O/Tetrahydrofuran: 0-30 %).
Ausbeute: 1,04 g (98 % d. Th.) rotbraunes Pulver
Wassergehalt: 6,9 %

| Elementaranalyse (berechnet auf wasserfreie Substanz): | | | | | | | |
|---|---|---|---|---|---|---|---|
| ber. | C 41,67 | H 4,17 | N 10,97 | Gd 17,60 | Fe 3,13 | Cl 1,98 | Na 2,61 |
| gef. | C 41,48 | H 4,32 | N 10,80 | Gd 17,43 | Fe 3,07 | Cl 1,78 | Na 2,38 |

### Beispiel 2

### a) Acetato [7, 12-diethyl-3, 8, 13, 17-tetramethylporphyrin-2, 18-dipropionylhydrazinato (2-)-K N21, K N22, K N23, K N24]-Mangan

1190 mg (2 mmol) 3,3'-(7, 12-Diethyl-3, 8, 13, 17-tetramethylporphyrin-2,18-diyl)-di(propanohydrazid), hergestellt analog zu H. Fischer, E. Haarer und F. Stadler, Z. Physiol. Chem. 241, 209; (1936), und 704,5 mg (2 mmol) Mangan(III)-acetylacetonat-Dihydrat werden in 150 ml Essigsäure/100 ml Chloroform 5 Stunden auf 80°C erhitzt. Dann wird im Vakuum eingedampft, der Rückstand in Wasser aufgeschlämmt, abfiltriert und mit Wasser gewaschen. Das getrocknete Rohprodukt wird aus Pyridin/ Diethylether umkristallisiert.
Ausbeute: 1,29 g (91 % d. Th.) rotbraunes Pulver

| Elementaranalyse: | | | | |
|---|---|---|---|---|
| ber. | C 61,18 | H 6,13 | N 15,86 | Mn 7,77 |
| gef. | C 61,03 | H 6,29 | N 15,75 | Mn 7,58 |

### b) Acetato [7, 12-diethyl-3, 8, 13,17-tetramethyl-2,18-bis {3, 6, 18-trioxo-8, 11, 14-tris(carboxymethyl) 17-oxa-4, 5, 8, 11, 14-pentaazanonadec-1-yl}porphyrinato(3-)]-Mangan

806,8 mg (2 mmol) 3-Ethoxy-carbonylmethyl-6-[2-(2,6-dioxomorpholino)ethyl]-3,6-diazaoctandi-säure (DTPA-monoethylester-monoanhydrid) werden in 250 ml absolutem Dimethylformamid suspendiert. Man überschichtet mit Stickstoff, setzt 1,0 g (10 mmol) Triethylamin und 706 mg (1 mmol) der Titelverbindung aus Beispiel 2a zu und rührt das resultierende Reaktionsgemisch 3 Tage bei Raumtemperatur. Nach beendeter Reaktion wird filtriert, das Lösungsmittel im Vakuum abgezogen und das verbleibende Öl mit 500 ml Diethylether verrieben. Der ausgefallene Feststoff wird abfiltriert und mit Diethylether und n-Hexan gewaschen. Zur Reinigung wird an Kieselgel RP-18 chromatographiert
(Eluens: H₂O/Tetrahydrofuran: 0-30 %).
Ausbeute: 1,35 g (89 % d. Th.) rotbraunes Pulver
Wassergehalt: 5,9 %

| Elementaranalyse (berechnet auf wasserfreie Substanz): | | | | |
|---|---|---|---|---|
| ber. | C 53,96 | H 6,19 | N 12,96 | Mn 3,63 |
| gef. | C 53,83 | H 6,34 | N 12,81 | Mn 3,49 |

### c) Chloro [7, 12-diethyl-3, 8, 13, 17-tetramethyl-2, 18-bis{3, 6, 18-trioxo-8, 11, 14-tris(carboxymethyl)-4, 5, 8, 11, 14-pentaazahexadecanoato}porphinato(3-)]-Mangan

1,31 g (0,865 mmol) des unter Beispiel 2b hergestellten Liganden werden in 400 ml Wasser gelöst. Durch Zugabe von 10 molarer wäßriger Natronlauge wird pH 13 eingestellt und 5 Stunden bei Raumtemperatur gerührt. Nach vollzogener Verseifung der Estergruppen wird mit konzentrierter Salzsäure pH 3 eingestellt. Man dampft im Vakuum zur Trockne ein. Der Rückstand wird an Kieselgel RP 18 chromatographiert (Eluens: H₂O/Tetrahydrofuran/Gradient).
Ausbeute: 1,15 g (93 % d. Th.) rotbraunes Pulver
Wassergehalt: 7,2 %

| Elementaranalyse (berechnet auf wasserfreie Substanz): | | | | | |
|---|---|---|---|---|---|
| ber. | C 51,93 | H 5,76 | N 13,68 | Mn 3,83 | Cl 2,47 |
| gef. | C 51,81 | H 5,93 | N 13,49 | Mn 3,70 | Cl 2,32 |

### d) {mu-[{16, 16'-[Chloromangan(III)-7, 12-diethyl-3, 8, 13, 17-tetramethylporphyrin-2, 18-diyl]-bis[3, 6, 9-tris(carboxymethyl)-11, 14-dioxo-3, 6, 9, 12, 13-pentaazahexadecanoato]}(8-)]}-digadolinato(2-),-Dinatrium

1,12 g (0,781 mmol)) des unter Beispiel 2c hergestellten Liganden werden in 400 ml Wasser gelöst und abwechselnd portionsweise 411,8 mg (1,56 mmol) Gadoliniumchlorid und 2n wäßrige Natronlauge zugesetzt, so daß der pH-Wert des Reaktionsgemisches stets zwischen 6,8 und 7,2 pendelt. Ist alles Gadoliniumchlorid zugesetzt, wird über Nacht bei Raumtemperatur nachgerührt. Zur Aufarbeitung wird das Lösungsmittel im Vakuum abgezogen und der Rückstand an Kieselgel RP-18 chromatographiert (Eluens: H₂O/Tetrahydrofuran: 0-30 %).
Ausbeute: 1,35 g (97 % d. Th.) rotbraunes Pulver
Wassergehalt: 6,5 %

| Elementaranalyse (berechnet auf wasserfreie Substanz): | | | | | | | |
|---|---|---|---|---|---|---|---|
| ber. | C 41,69 | H 4,18 | N 10,98 | Gd 17,61 | Mn 3,08 | Cl 1,98 | Na2,57 |
| gef. | C 41,48 | H 4,33 | N 10,81 | Gd 17,50 | Mn 2,89 | Cl 1,85 | Na 2,34 |

### Beispiel 3

### a) Acetato[7, 12-diethyl-3, 8,13, 17-tetramethylporphyrin-2,18-dipropionylhydrazinato(2-)-K N21, K N22, K N23, K N24]-Kobalt

1190 mg (2 mmol) 3,3'-(7, 12-Diethyl-3, 8, 13, 17-tetramethylporphyrin-2,18-diyl)-di(propanohydrazid), hergestellt analog zu H. Fischer, E. Haarer und F. Stadler, Z. Physiol. Chem. 241, 209 (1936), und 712,52 mg (2 mmol) Kobalt(III)-acetylacetonat werden in 150 ml Essigsäure/100 ml Chloroform 5 Stunden auf 80°C erhitzt. Dann wird im Vakuum eingedampft, der Rückstand in Wasser aufgeschlämmt, abfiltriert und mit Wasser gewaschen. Das getrocknete Rohprodukt wird
aus Pyridin/ Diethylether umkristallisiert.
Ausbeute: 1,31 g (92 % d. Th.) rotbraunes Pulver

| Elementaranalyse: | | | | |
|---|---|---|---|---|
| ber. | C 60,84 | H 6,10 | N 15,77 | Co 8,29 |
| gef. | C 60,71 | H 6,29 | N 15,58 | Co 8,14 |

### b) Acetato[7, 12-diethyl-3, 8, 13, 17-tetramethyl-2, 18-bis{3, 6, 18-trioxo-8, 11, 14-tris(carboxymethyl)-17-oxa-4, 5, 8, 11, 14-pentaazanonadec-1-yl}porphinato(3-)]-Kobalt

806,8 mg (2 mmol) 3-Ethoxy-carbonylmethyl-6-[2-(2,6-dioxomorpholino)ethyl]-3,6-diaza octandi-säure (DTPA-monoethylester-monoanhydrid) werden in 250 ml absolutem Dimethylformamid suspendiert. Man überschichtet mit Stickstoff, setzt 1,0 g (10 mmol) Triethylamin und 710 mg (1 mmol) der Titelverbindung aus Beispiel 3a zu und rührt das resultierende Reaktionsgemisch 3 Tage bei Raumtemperatur. Nach beendeter Reaktion wird filtriert, das Lösungsmittel im Vakuum abgezogen und das verbleibende Öl mit 500 ml Diethylether verrieben. Der ausgefallene Feststoff wird abfiltriert und mit Diethylether und n-Hexan gewaschen. Zur Reinigung wird an Kieselgel RP-18 chromatographiert (Eluens: H₂O/Tetrahydrofuran: 0-30 %).
Ausbeute: 1,32 g (87 % d. Th.) rotbraunes Pulver
Wassergehalt: 6,7 %

| Elementaranalyse (berechnet auf wasserfreie Substanz): | | | | |
|---|---|---|---|---|
| ber. | C 53,82 | H 6,18 | N 12,92 | Co 3,88 |
| gef. | C 53,72 | H 6,35 | N 12,81 | Co 3,69 |

### c) Chloro [7, 12-diethyl-3, 8, 13, 17-tetramethyl-2, 18-bis{3, 6, 18-trioxo-8, 11, 14 tris(carboxymethyl)-4, 5, 8, 11, 14-pentaazahexadecanoato}porphinato(3-)]-Kobalt

1,28 g (0,844 mmol) des unter Beispiel 3b hergestellten Liganden werden in 400 ml Wasser gelöst. Durch Zugabe von 10 molarer wäßriger Natronlauge wird pH 13 eingestellt und 5 Stunden bei Raumtemperatur gerührt. Nach vollzogener Verseifung der Estergruppen wird mit konzentrierter Salzsäure pH 3 eingestellt. Man dampft im Vakuum zur Trockne ein. Der Rückstand wird an Kieselgel RP 18 chromatographiert (Eluens: H₂O/Tetrahydrofuran/Gradient).
Ausbeute: 1,15 g (95 % d. Th.) rotbraunes Pulver
Wassergehalt: 4,9 %

| Elementaranalyse (berechnet auf wasserfreie Substanz): | | | | | |
|---|---|---|---|---|---|
| ber. | C 51,79 | H 5,75 | N 13,64 | Co 4,10 | Cl 2,47 |
| gef. | C 51,60 | H 5,89 | N 13,51 | Co 3,97 | Cl 2,35 |

### d) {mu-[{16,16'-[Chlorokobolt(III)-7,12-diethyl-3, 8, 13, 17-tetramethylporphyrin-2,18-diyl]-bis[3, 6, 9-tris(carboxymethyl)-11,14-dioxo-3, 6, 9, 12, 13-pentaazahexadecanoato]}(8-)]}-digadolinato(2-), Dinatrium

1,12 g (0,779 mmol) des unter Beispiel 3c hergestellten Liganden werden in 400 ml Wasser gelöst und abwechselnd portionsweise 410,6 mg (1,59 mmol) Gadoliniumchlorid und 2n wäßrige Natronlauge zugesetzt, so daß der pH-Wert des Reaktionsgemisches stets zwischen 6,8 und 7,2 pendelt. Ist alles Gadoliniumchlorid zugesetzt, wird über Nacht bei Raumtemperatur nachgerührt. Zur Aufarbeitung wird das Lösungsmittel im Vakuum abgezogen und der Rückstand an Kieselgel RP-18 chromatographiert
(Eluens: H₂O/Tetrahydrofuran: 0-30 %).
Ausbeute: 1,34 g (96 % d. Th.) rotbraunes Pulver
Wassergehalt: 7,8 %

| Elementaranalyse (berechnet auf wasserfreie Substanz): | | | | | | | |
|---|---|---|---|---|---|---|---|
| ber. | C 41,59 | H 4,17 | N 10,95 | Gd 17,57 | Co 3,29 | Cl 1,98 | Na 2,57 |
| gef. | C 41,48 | H 4,32 | N 10,84 | Gd 17,43 | Co 3,14 | Cl 1,81 | Na 2,31 |

### Beispiel 4

### a) [7, 12-diethyl-3, 8, 13, 17-tetramethylporphyrin-2,18-dipropionylhydrazinato (2-)-K N21, K N22, K N23, K N24]-Kupfer

1190 mg (2 mmol) 3,3'-(7, 12-Diethyl-3, 8, 13, 17-tetramethylporphyrin-2,18-diyl)-di(propanohydrazid), hergestellt analog zu H. Fischer, E. Haarer und F. Stadler, Z. Physiol. Chem. 241,209(1936), und 523,5 mg (2 mmol) Kupfer(II)-acetylacetonat werden in 150 ml Essigsäure/100 ml Chloroform 5 Stunden auf 80°C erhitzt. Dann wird im Vakuum eingedampft, der Rückstand in Wasser aufgeschlämmt, abfiltriert und mit Wasser gewaschen. Das getrocknete Rohprodukt wird aus Pyridin/ Diethylether umkristallisiert.
Ausbeute: 1,19 g (91 % d. Th.) rotbraunes Pulver

| Elementaranalyse: | | | | |
|---|---|---|---|---|
| ber. | C 62,22 | H 6,14 | N 17,07 | Cu 9,68 |
| gef. | C 62,10 | H 6,33 | N 16,92 | Cu 9,51 |

### b) [7, 12-Diethyl-3, 8, 13, 17-tetramethyl-2, 18-bis{3, 6, 18-trioxo-8, 11, 14-tris(carboxymethyl)-17-oxa-4, 5, 8, 11, 14-pentaazanonadec-1-yl}-porphinato (2-)]-Kupfer

806,8 mg (2 mmol) 3-Ethoxy-carbonylmethyl-6-[2-(2,6-dioxomorpholino)ethyl]-3,6-diaza octandi-säure (DTPA-monoethylester-monoanhydrid) werden in 250 ml absolutem Dimethylformamid suspendiert. Man überschichtet mit Stickstoff, setzt 1,01 g (10 mmol) Triethylamin und 656 mg (1 mmol) der Titelverbindung aus Beispiel 4a zu und rührt das resultierende Reaktionsgemisch 3 Tage bei Raumtemperatur. Nach beendeter Reaktion wird filtriert, das Lösungsmittel im Vakuum abgezogen und das verbleibende Öl mit 500 ml Diethylether verrieben. Der ausgefallene Feststoff wird abfiltriert und mit Diethylether und n-Hexan gewaschen. Zur Reinigung wird an Kieselgel RP-18 chromatographiert (Eluens: H₂O/Tetrahydrofuran: 0-30 %).
Ausbeute: 1,27 g (87 % d. Th.) rotbraunes Pulver
Wassergehalt: 6,2 %

| Elementaranalyse (berechnet auf wasserfreie Substanz): | | | | |
|---|---|---|---|---|
| ber. | C 54,18 | H 6,20 | N 13,40 | Cu 4,34 |
| gef. | C 54,02 | H 6,31 | N 13,29 | Cu 4,18 |

### c) [7, 12-Diethyl-3, 8, 13, 17-tetramethyl-2, 18-bis{3, 6, 18-trioxo-8, 11, 14-tris(carboxymethyl)-4, 5, 8, 11, 14-pentaazahexadecanoato}porphinato (2-)]-Kupfer

1,24 g (0,848 mmol) des unter Beispiel 4b hergestellten Liganden werden in 400 ml Wasser gelöst. Durch Zugabe von 10 molarer wäßriger Natronlauge wird pH 13 eingestellt und 5 Stunden bei Raumtemperatur gerührt. Nach vollzogener Verseifung der Estergruppen wird mit konzentrierter Salzsäure pH 3 eingestellt. Man dampft im Vakuum zur Trockne ein. Der Rückstand wird an Kieselgel RP 18 chromatographiert (Eluens: H₂O/Tetrahydrofuran/Gradient).
Ausbeute: 1,15 g (96 % d. Th.) rotbraunes Pulver
Wassergehalt: 4,4 %

| Elementaranalyse (berechnet auf wasserfreie Substanz): | | | | |
|---|---|---|---|---|
| ber. | C 52,93 | H 5,87 | N 13,94 | Cu 4,52 |
| gef. | C 52,83 | H 6,04 | N 13,85 | Cu 4,38 |

### d) {mu-[{16,16'-[Kupfer(II)-7, 12-diethyl-3, 8, 13, 17-tetramethylporphyrin-2,18-diyl]-bis[3, 6, 9-tris(carboxymethyl)-11, 14-dioxo-3, 6, 9, 12, 13-pentaazahexadecanoato]}(8-)]}-digadolinato(2-), Dinatrium

1,12 g (0,796 mmol) des unter Beispiel 4c hergestellten Liganden werden in 400 ml Wasser gelöst und abwechselnd portionsweise 420 mg (1,59 mmol) Gadoliniumchlorid und 2n wäßrige Natronlauge zugesetzt, so daß der pH-Wert des Reaktionsgemisches stets zwischen 6,8 und 7,2 pendelt. Ist alles Gadoliniumchlorid zugesetzt, wird über Nacht bei Raumtemperatur nachgerührt. Zur Aufarbeitung wird das Lösungsmittel im Vakuum abgezogen und der Rückstand an Kieselgel RP-18 chromatographiert
(Eluens: H₂O/Tetrahydrofuran: 0-30 %).
Ausbeute: 1,37 g (98 % d. Th.) rotbraunes Pulver
Wassergehalt: 7,6 %

| Elementaranalyse (berechnet auf wasserfreie Substanz): | | | | | | |
|---|---|---|---|---|---|---|
| ber. | C 42,32 | H 4,24 | N 11,15 | Gd 17,88 | Cu 3,61 | Na 2,61 |
| gef. | C 42,18 | H 4,38 | N 11,09 | Gd 17,70 | Cu 3,48 | Na 2,47 |

### Beispiel 5

### a) Acetato {7, 12-diethyl-3, 8, 13, 17-tetramethyl-2,18-bis[15, 15-dimethyl-3, 6, 13-trioxo-8-(2-{N, N-bis[(t butoxycarbonyl)methyl]amino}-ethyl)-11-[(t butoxycarbonyl)-methyl]14-oxa-4,5, 8, 11-tetraazahexadec-1-yl}-porphyrinato(3-) Mangan

8,31 g (13,45 mmol) 3,9-Bis(t butoxycarbonyl)methyl]-6-carboxymethyl-3,6,9-triazaundecandisäure-di-t.butylester, und 2,09 g (15 mmol) 4-Nitrophenol werden in 60 ml Dimethylformamid gelöst und bei 0°C 5,16 g (25 mmol) N,N'-Dicyclohexylcarbodiimid zugegeben. Man rührt 3 Stunden bei 0°C, dann über Nacht bei Raumtemperatur. Zu der so hergestellten Aktivester-Lösung tropft man 2,37 g (3,36 mmol) der Titelverbindung aus Beispiel 2a, (gelöst in 50 ml Pyridin) zu und rührt über Nacht. Man gibt 100 ml einer 5 %igen aqu. Ammoniumchlorid-Lösung zu, dampft im Vakuum zur Trockne ein und chromatographiert den Rückstand an Kieselgel (Laufmittel: Dichlormethan/2-Propanol= 20:1).
Ausbeute: 5,25 g (83 % d. Th.) eines dunkelbraunen Feststoffes

| Elementaranalyse: | | | | | |
|---|---|---|---|---|---|
| ber. | C 59,97 | H 7,82 | N 10,42 | Mn 2,92 | Cl 1,88 |
| gef. | C 59,83 | H 8,03 | N 10,28 | Mn 2,83 | Cl 1,67 |

### b) {mu-[Acetatomangan(III)-{13,13'-[7,12-diethyl-3,8,13,17-tetramethylporphyrin-2, 18-diyl]-bis{3-carboxymethyl-6-(2-{N, N-bis[(carboxy)methyl] amino}ethyl)-8, 11-dioxo-3, 6, 9, 10-tetraazatridecanoato]}(8-)]}digadolinato(2-), Dinatrium

5,25 g (2,79 mmol) der Titelverbindung aus Beispiel 5a werden in 100 ml Trifluoressigsäure gelöst und 8 Stunden bei Raumtemperatur gerührt. Man dampft im Vakuum zur Trockne ein. Der so erhaltene Ligand wird in 100 ml Wasser gelöst und 1,01 g (2,79 mmol) Gadoliniumoxid zugegeben. Man rührt bei 60 °C und hält durch Zugabe von 1 N aqu. Natronlauge den pH-Wert bei 5. Die Lösung wird filtriert und das Filtrat mit 1 N aqu. Natronlauge auf pH 7,2 gestellt. Anschließend wird an RP 18 chromatographiert (Laufinittel: Gradient aus Wasser/Acetonitril).
Ausbeute: 4,63 g (93 % d. Th.) eines braunen, amorphen Feststoffes
Wassergehalt: 9,2 %

| Elementaranalyse (berechnet auf wasserfreie Substanz): | | | | | | | |
|---|---|---|---|---|---|---|---|
| ber. | C 41,69 | H 4,18 | N 10,98 | Mn 3,08 | Cl 1,98 | Gd 17,61 | Na 2,57 |
| gef. | C 41,54 | H 4,35 | N 10,82 | Mn 2,91 | Cl 1,85 | Gd 17,45 | Na 2,34 |

### Beispiel 6

### a) {10, 10'-(my-Acetatomangan(III)-{10, 10'-(7, 12-diethyl-3, 8, 13, 17-tetramethylporphyrin-2, 18-diyl)bis[(1RS)-1-methyl-2, 5, 8-trioxo-3, 6, 7-triaza-dec-1-yl]}) bis[1, 4, 7, 10-tetraazacyclododecan-1, 4, 7-triacetato(3-)]}digadolinium

8,47 g (13,45 mmol) des Gd-Komplexes der 10-(4-Carboxy-2-oxo-3-aza-1-methylbutyl)-1,4,7,10-tetraazacyclododecan-1,4,7-triessigsäure, 0,64 g Lithiumchlorid (15 mmol) und 2,09 g (15 mmol) 4-Nitrophenol werden in 100 ml Dimethylsulfoxid bei 50°C gelöst. Nach Abkühlen auf Raumtemperatur werden 5,16 g (25 mmol) N,N'Dicyclohexylcarbodiimid zugesetzt und 12 Stunden voraktiviert. Zu der so hergestellten Lösung gibt man 2,37 g (3,36 mmol) der Titelverbindung aus Beispiel 2a 0,71 g (7 mmol) Triethylamin und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylhamstoff abfiltriert und das Filtrat an RP 18 chromatographiert (Laufmittel: Gradient aus Tetrahydrofuran/Wasser).
Ausbeute: 5,06 g (79 % d. Th.) eines dunkelbraunen amorphen Pulvers
Wassergehalt: 8,3 %

| Elementaranalyse (berechnet auf wasserfreie Substanz): | | | | | | |
|---|---|---|---|---|---|---|
| ber. | C 45,36 | H 5,08 | N 13,22 | Gd 16,50 | Mn 2,88 | Cl 1,86 |
| gef. | C 45,24 | H 5,21 | N 13,13 | Gd 16,38 | Mn 2,71 | Cl 1,72 |

### Beispiel 7

### a) Konjugat aus Acetato [7, 12-diethyl-3, 8, 13, 17-tetramethylporphyrin-2, 18-dipropionylhydrazinato (2-)- K N21, K N22, K N23, K N24]-Eisen und dem 10-[7-(4-Isothiocyanatophenyl)-2-hydroxy-5-oxo-7-(carboxymethyl)-4-aza-heptyl)]-1,4,7-tris(carboxylatomethyl)-1,4,7-tris(carboxylatomethyl)-1,4,7,10-tetraazacyclododecan, Gadoliniumkomplex, Natriumsalz {10,10'-{my-Chloroeisen(III){10,10'-[(7, 12-diethyl-3, 8, 13, 17-tetramethylporphyrin-2, 18-diyl)bis{(1-oxopropan-3, 1-diyl)hydrazino-thiocarbonylamino-4, 1-phenylen[(3RS)-3-carboxymethyl-1-oxopropan-3,1-diyl]amino(2-hydroxypropan-3, 1-diyl)}]}bis[1, 4, 7, 10-tetraazacyclododecan-1, 4, 7-triacetato (4-)]}digadolinium, Dinatrium)

Zu 708 mg (1 mmol) der Titelverbindung aus Beispiel 1a und 1806 mg (2,2 mmol) in 50 ml Wasser gibt man 1,01 g (10 mmol) Triethylamin und rührt 12 Stunden bei Raumtemperatur. Man dampft im Vakuum zur Trockne ein und chromatographiert an Kieselgel (Laufinittel: Methanol/Wasser/Eisessig= 10/5/1). Die produkthaltigen Fraktionen werden zur Trockne eingedampft, der Rückstand in 100 ml Wasser gelöst und mit 2 N Natronlauge auf pH 7,2 gestellt. Anschließend wird gefriergetrocknet.
Ausbeute: 2,11 g (92 % d. Th.) eines dunkelbraunen, amorphen Pulvers
Wassergehalt: 8,5 %

| Elementaranalyse (berechnet auf wasserfreie Substanz): | | | | | | | | |
|---|---|---|---|---|---|---|---|---|
| ber. | C 44,95 | H 5,09 | N 12,19 | Cl 1,54 | Fe 2,43 | S 2,79 | Gd 13,69 | Na 2,00 |
| gef. | C 44,83 | H 5,19 | N 12,03 | Cl 1,38 | Fe 2,38 | S 2,58 | Gd 13,48 | Na 1,78 |

### Beispiel 8

### Herstellung einer Formulierung des {mu-[{16,16'-[Chloroeisen(III)-7, 12-diethyl-3, 8, 13,17-tetramethylporphyrin-2, 18-diyl]-bis[3, 6, 9-tris(carboxymethyl)-11, 14-dioxo-3, 6, 9, 12, 13-pentaazahexadecanoato]}(8-)]}-digadolinato(2-), Dinatrium (Zusatz von Mannitol)

50 mmol der Titelverbindung aus Beispiel 1d, 10 mmol Tris-Puffer (Tris(hydroxymethyl)aminomethan Salzsäure pH 7,4) und 120 mmol Mannitol werden in 500 ml bidestilliertem Wasser gelöst und im Meßkolben mit Wasser auf 1 Volumen aufgefüllt. Die so erhaltene Lösung wird über eine 0,2 µm Membran filtriert und in Vials abgefüllt. Eine so hergestellte Lösung kann direkt für die NMR-Diagnostik verwendet werden.

### Beispiel 9

### Herstellung einer Formulierung des {mu-[{16, 16'-[Chloromangan(III)-7, 12-diethyl-3, 8, 13, 17-tetramethylporphyrin-2, 18-diyl]-bis[3, 6, 9-tris(carboxymethyl)-11, 14-dioxo-3, 6, 9,12, 13-pentaazahexadecanoato]}(8-)]}-digadolinato(2-),-Dinatrium (Zusatz von Kochsalz)

10 mmol der Titelverbindung aus Beispiel 2d, 10 mmol Tris-Puffer (Tris(hydroxymethyl)aminomethan Salzsäure pH 7,4) und 60 mmol Natriumchlorid werden in 500 ml bidestilliertem Wasser gelöst und im Meßkolben mit Wasser auf 11 Volumen aufgefüllt. Die so erhaltene Lösung wird über eine 0,2 um Membran filtriert und in Vials abgefüllt. Eine so hergestellte Lösung kann direkt für die NMR-Diagnostik verwendet werden.

### Beispiel 10

### MRI-Experimente an Tieren mit induziertem Myocardinfarkt

Das nekroseselektive Enhancement wurde nach einmaliger intravenöser Applikation der Substanz aus Beispiel 1d an Tieren mit experimentell erzeugtem Myocardinfarkt im MRI Experiment untersucht. Die Induktion der Herzinfarkte erfolgte an narkotisierten (Domitor®/Dormicum®, i.m.) Ratten (Han.Wistar, Schering SPF, ca.300 g KGW) durch Okklusion der linken Coronararterie. Die Kontrastmittelapplikation (Dosis: 100 µmol Gd / kg KGW) erfolgte ca. 24 h nach der Infarktinduktion. Die Tiere wurden vor und bis 24 h nach KM-Applikation MR-tomographisch (SISCO SIS 85, 2 Tesla; SE-Sequenz, EKG-getriggert, T_{R}: ca. 400 ms, T_{E}: 10 ms, nt=4, ni=256, FOV: 7*7 cm, SD ≈ 3 mm, je 1 Schicht axial) untersucht. Ca. 24 h p. i. wurden die Tiere - im MRT - durch eine Narkotikaüberdosis getötet und zusätzlich ein MRI-Experiment am "frischtoten" Tier (--> keine Bewegungsartefakte) durchgeführt. Zur Verifizierung des Infarktes (Größe und Lage) wurde das Herz präpariert, in Scheiben geschnitten und anschließend eine NBT ("Vital-") Färbung durchgeführt. Vor KM-Applikation ist das infarzierte Areal nicht vom "normalen" Myocard zu unterscheiden, da sich beide Areale isointens darstellen (s. Abb.: 1a). Direkt nach der Substanzapplikation stellt sich der nicht-perfundierte Anteil des Myocards als hypointenses Areal dar (s. Abb.: 1b). Ab ca. 30 min p. i. steigt die Signalintensität im nicht-perfundierten Areal etwas an bzw. die Größe des abgegrenzten (Signalarmen) Areals nimmt ab (--> langsame Diffusion in die Nekrose). In der mittleren und v. a. späten Phase (ab 2.5 bis ca. 24 h p. i.) ist ein deutliches Enhancement im nekrotischen Areal des Myocards festzustellen (s. Abb.: 1c, d, e). Die Abgrenzung des nekrotischen Areals im MRI-Experiment korreliert sehr gut mit den Ergebnissen der histologischen "Vital"-Färbung.

### Beispiel 11

### MRI-Experimente an Tieren mit induziertem Myocardinfarkt

Das nekroseselektive Enhancement wurde nach einmaliger intravenöser Applikation der Substanz aus Beispiel 2d an Tieren mit experimentell erzeugtem Myocardinfarkt im MRI Experiment untersucht. Die Induktion der Herzinfarkte erfolgte an narkotisierten (Domitor®/Dormicum®, i.m.) Ratten (Han.Wistar, Schering SPF, ca.300 g KGW) durch Okklusion der linken Coronararterie. Die Kontrastmittelapplikation (Dosis: 100 µmol Gd / kg KGW ) erfolgte ca. 24 h nach der Infarktinduktion. Die Tiere wurden vor und bis 3 h (kontinuierlich) sowie 24 h nach KM-Applikation MR-tomographisch (SISCO SIS 85, 2 Tesla; SE-Sequenz, EKG-getriggert, T_{R}: ca. 400 ms, T_{E}: 10 ms, nt=4, ni=256, FOV: 7*7 cm, SD ≈ 3 mm, je 1 Schicht axial) untersucht. Ca. 24 h p. i. wurden die Tiere - im MRT - durch eine Narkotikaüberdosis getötet und ein MRI-Experiment am "frischtoten" Tier (--> keine Bewegungsartefakte) durchgeführt. Zur Verifizierung des Infarktes (Größe und Lage) wurde das Herz präpariert, in Scheiben geschnitten und anschließend eine NBT (nitro blue tetrazolium chlorid) Färbung durchgeführt. Vor KM-Applikation ist das infarzierte Areal nicht vom "normalen" Myocard zu unterscheiden, da sich beide Areale isointens darstellen (s. Abb.: 2a). Direkt nach der Substanzapplikation stellt sich der nicht-perfundierte Anteil des Myocards als hypointenses Areal dar (s. Abb.: 2b). Ab ca. 30 min p. i. steigt die Signalintensität im nicht-perfundierten Areal etwas an bzw. die Größe des abgegrenzten (signalarmen) Areals nimmt ab (--> langsame Diffusion in die Nekrose). In der mittleren und v. a. späten Phase (ab 2.5 bis ca. 24 h p. i.) ist ein deutliches Enhancement im nekrotischen Areal des Myocards festzustellen (s. Abb.: 2c, 2d). Die Abgrenzung des nekrotischen Areals im MRI-Experiment korreliert sehr gut mit den Ergebnissen der histologischen NBT ("Vital"-) Färbung.

### Beispiel 12

### MRI-Experimente an Tieren mit induziertem Myocardinfarkt

Das nekroseselektive Enhancement wurde nach einmaliger intravenöser Applikation der Substanz aus Beispiel 4d an Tieren mit experimentell erzeugtem Myocardinfarkt im MRI Experiment untersucht. Die Induktion der Herzinfarkte erfolgte an narkotisierten (Domitor®/Dormicum®, i. m.) Ratten (Han. Wistar, Schering SPF, ca. 300 g KGW) durch Okklusion der linken Coronararterie. Die Kontrastmittelapplikation (Dosis: 100 µmol Gd / kg KGW) erfolgte ca. 24 h nach der Infarktinduktion. Die Tiere wurden vor und bis 3 h (kontinuierlich) sowie 24 h nach KM-Applikation MR-tomographisch (SISCO SIS 85, 2 Tesla; SE-Sequenz, EKG-getriggert, T_{R}: ca. 400 ms, T_{E}: 10 ms, nt=4, ni=256, FOV: 7*7 cm, SD ≈ 3 mm, je 1 Schicht axial) untersucht. Ca. 24 h p. i. wurden die Tiere - im MRT - durch eine Narkotikaüberdosis getötet und ein MRI-Experiment am "frischtoten" Tier (--> keine Bewegungsartefakte) durchgeführt. Zur Verifizierung des Infarktes (Größe und Lage) wurde das Herz präpariert, in Scheiben geschnitten und anschließend eine NBT (nitro blue tetrazolium chlorid) Färbung durchgeführt. Vor KM-Applikation ist das infarzierte Areal nicht vom "normalen" Myocard zu unterscheiden, da sich beide Areale isointens darstellen (s. Abb.: 3a). Direkt nach der Substanzapplikation stellt sich der nicht-perfundierte Anteil des Myocards als hypointenses Areal dar (s. Abb.: 3b). Ab ca. 30 min p. i. steigt die Signalintensität im nicht-perfundierten Areal etwas an bzw. die Größe des abgegrenzten (signalarmen) Areals nimmt ab (--> langsame Diffusion in die Nekrose). In der mittleren und v. a. späten Phase (ab 2.5 bis ca. 24 h p. i.) ist ein deutliches Enhancement im nekrotischen Areal des Myocards festzustellen (s. Abb.: 3c, 3d). Die Abgrenzung des nekrotischen Areals im MRI-Experiment korreliert sehr gut mit den Ergebnissen der histologischen NBT ("Vital"-) Färbung.

### Beispiel 13

### Einwirkung von Licht (ED₅₀) auf eine Tumorzellkultur in Gegenwart von Porphyrinen

In Kulturflaschen von 25 ml Inhalt wird eine Zellkultur von humanem Coloncarcinom (HT-29 P9) 3 Tage bei 37°C vermehrt. Die Kulturen werden in zwei Grupen aufgeteilt und mit Lösungen der Prüfsubstanzen (50 mmol Porphyrineinheit (PE) /l, verdünnt mit fötalem Kälberserum) in zunehmender Menge (0; 1,5; 5; 8,5; 12; 15,5; 19 µmol PE/l) versetzt. Die Proben werden drei Tage lang mit einer Xenon-Lampe (8,5 k Lux, UV-Filter) bestrahlt. Die erste Gruppe erhält täglich 2 Bestrahlungen von je 30 Minuten Dauer im Abstand von 4 Stunden. Die restliche Zeit bleibt sie im Dunkeln im Brutschrank. Die zweite Gruppe wird nicht belichtet und bleibt die gesamte Zeit im Dunkeln im Brutschrank. Am vierten Tag wird das Zellwachstum durch Lebend-Tot-Färbung und Zählung mit der Zählkammer bestimmt.

In der Tabelle ist die Konzentration angegeben, bei der etwa die Hälfte der Zellen nicht mehr vital ist.

| Verbindung | ED₅₀ [mmol PE/l] |
|---|---|
| I | > 1,25 |
| II | > 1,25 |
| III | > 1,25 |
| IV | 6,4·10⁻³ |
| I Verbindung aus Beispiel 1d | |
| II Verbindung aus Beispiel 2d | |
| m Verbindung aus Beispiel 4d | |
| IV Verbindung aus Beispiel 1c, DE 423 2925 | |
| IV: des {mu-[{16, 16'-(7, 12-Diethyl-3, 8, 13, 17-tetramethylporphyrin-2, 18-diyl]-bis[3,6,9-tris(carboxymethyl)-11, 14-dioxo-3, 6, 9,12, 13-pentaazahexadecanoato]}(8-)]}-digadolinato(2-),-Dinatrium | |

### Beispiel 14

Bestimmung der Verträglichkeit der Titelverbindung aus Beispiel 2d

Es soll die Akute Verträglichkeit der Titelverbindung aus Beispiel 2d nach einmaliger intravenöser Gabe an der Maus untersucht werden.

### Versuch:

- Testmethode:: Methode KM D1, 5. Auflage
- Tiermaterial:: SPF-Mäuse (NMRI, Schering), 18-22 g, f:m = 50:50
- Injektionsgeschwindigkeit:: 2 ml/min.
- Kriterium der Wirksamkeit:: exitus letalis
- Beobachtungszeitraum:: 7 Tage

Die Lebern und Nieren aller überlebenden Tiere waren unauffällig. Es ist eine LD50 [mmol Gd/kg] von ≥ 5 zu erwarten.

### Beispiel 15

Bestimmung der Relaxivity R1 [L·mmol⁻¹ ·s⁻¹]
Gerät: Minispec PC 20
Messung bei 40°C; 0,47 Tesla
T1-Sequenz: 180°-TI-90°, Inversion Recovery

| Substanz | Konz. im Ansatz [mmol/L] | Medium | R1 |
|---|---|---|---|
| 1d | 0.19-0.71 n=3 | Wasser | 11.9 ± 1.0 |
| 2d | 0.22 - 0.84 n=3 | Wasser | 9.3 ± 0.1 |
| 4d | 0.23 - 0.84 n = 3 | Wasser | 11.0 ± 0.1 |
| 1c, DE 4232925 | 0.05 - 0.5 n = 3 | Wasser | 9.2 ± 0.1 |

### Beispiel 16

### In-vivo-Vergleich, Titelverbindung aus Beispiel 1d mit Dy-DTPA hinsichtlich der Blutkinetik

Als Versuchstiere dienten drei 250 g schwere, männliche (Schering-SPF-) Ratten. Je Tier wurden 0.5 ml eines Kontrastmittel-Gemisches aus der Verbindung aus Beispiel 1d (45 mmol Gd/l), im folgenden Verbindung 1 genannt, und dem Dysprosium-Komplex der (Dy-DTPA, 57 mmol Dy/l), im folgenden Verbindung 2 genannt, intravenös appliziert. Die dadurch applizierte Dosis betrug 82 µmol Gd/kg (Verbindung 1), bzw. 103 µmol Dy/kg (Verbindung 2). Über einen Katheter in der Arteria carotis communis wurden Blutproben zu folgenden Zeitpunkten entnommen: 1, 3, 5, 10, 15, 20, 30, 45, 60, 90, 120 min p.i. In den gewonnenen Blutproben wurden jeweils parallel die Konzentrationen an Gadolinium (Gd) und Dysprosium (Dy) mittels Atomemissionsspektrometrie (ICP-AES) gemessen. Der im Blutraum verbliebene Anteil der injizierten Kontrastmittel Verbindung 1 (Gd) und Verbindung 2 (Dy, Vergleichssubstanz) kann durch die unterschiedliche Markierung im gleichen Tier verglichen werden. Aus den Blut- bzw. Plasmakonzentrationen kann mittels spezieller Software (Topfit-Programm) die α-t ½ und β- t ½ Eliminations-Halbwertzeiten, das Verteilungsvolumen sowie die total Clearance für Blut und Plasma errechnet werden. Damit liefern diese Daten Angaben über den Verbleib der Verbindungen im Intravasalraum, die Verteilungsverhältnisse im Organismus und die Eliminierung.

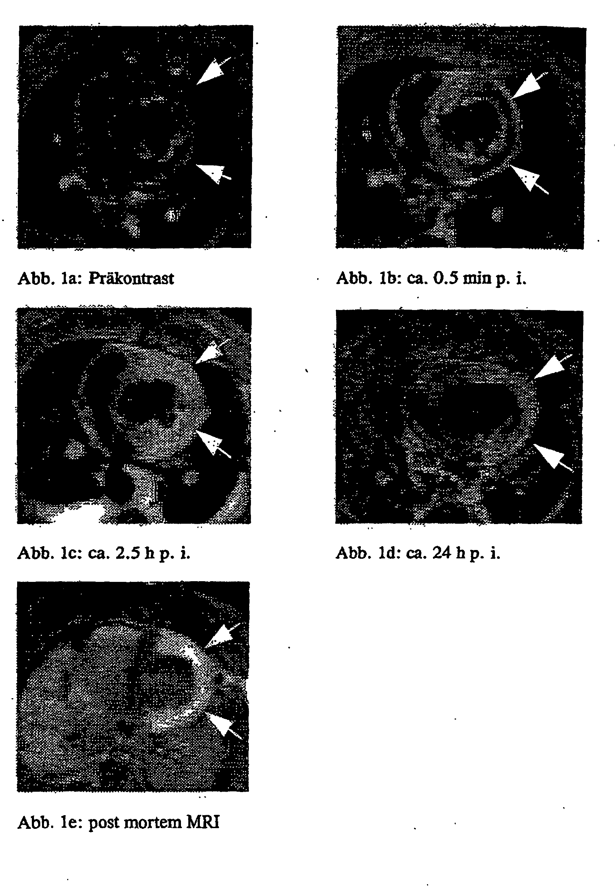

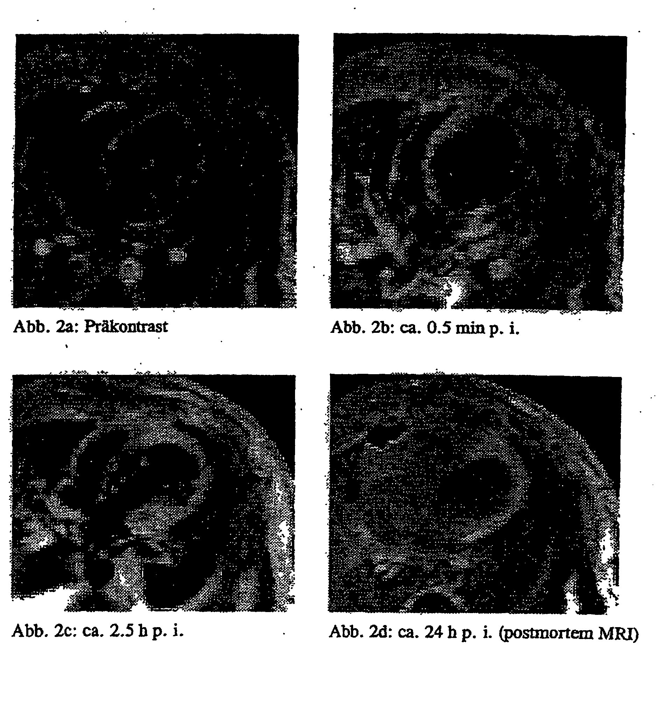

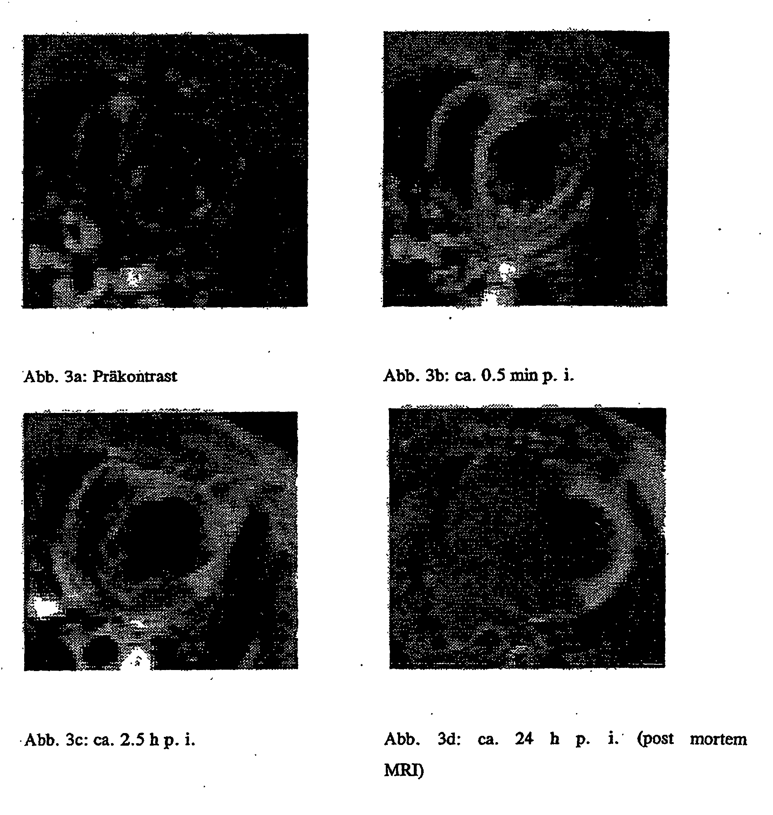

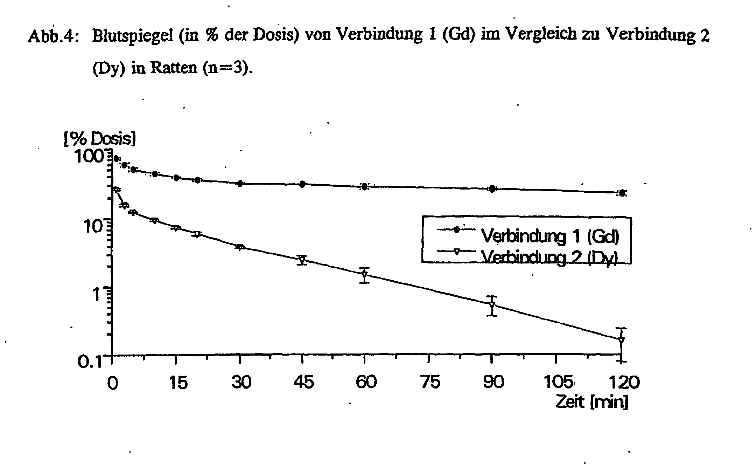

Ergebnisse: Die Bluteliminationskinetik von Verbindung 1 unterscheidet sich deutlich von der des extrazellulären Kontrastmittel (Verbindung 2) (siehe Abb. 4, Tab. 1). Die Blutkonzentrationen liegen nach drei Minuten noch bei 56-63 % der Dosis und nach 120 min bei 22-25 % der Dosis. Die Ausscheidung/Diffusion ins Gewebe ist deutlich verlangsamt ( -Halbwertzeit 173 min). Das Verteilungsvolumen und die Total Clearance sind ebenfalls deutlich kleiner verglichen zum Dy-DTPA, d.h. Verbindung 1 verteilt sich nicht wie Verbindung 2 im Intravasalraum (Gefäße) *und* im Extrazellularraum, sondern größtenteils *nur* im Intravasalraum (Blood-Pool-Eigenschaften dieses Porphyrins).

Die lange Blutverweildauer von Verbindung 1 deutet auf eine sehr hohe Plasmaproteinbindung. Damit verfügt die im Beispiel beschriebene Verbindung über die Erfordernisse an ein blood-pool-agent.

**Tab.1:**

| Pharmakokinetische Parameter (Plasma), sowie Eliminationshalbwertzeiten von Verbindung 1 im Vergleich zu Verbindung 2 in Ratten (n=3). | | | |
|---|---|---|---|
| | | **Verbindung 1** | **Verbindung 2** |
| **α-t½** | min | 3.56 ± 1.01 | 1.19 ± 0.24 |
| **β-t½** | min | 172.90 ± 38.76 | 18.64 ± 2.47 |
| **Vd ss** | L/kg | 0.08 ± 0.00 | 0.19 ± 0.01 |
| **Total Clearance** | ml/min*kg | 0.34 ± 0.07 | 7.74 ± 0.56 |

## Patentansprüche

1. Verwendung von mindestens einem Porphyrin-Komplex bestehend aus einem Liganden der allgemeinen Formel I 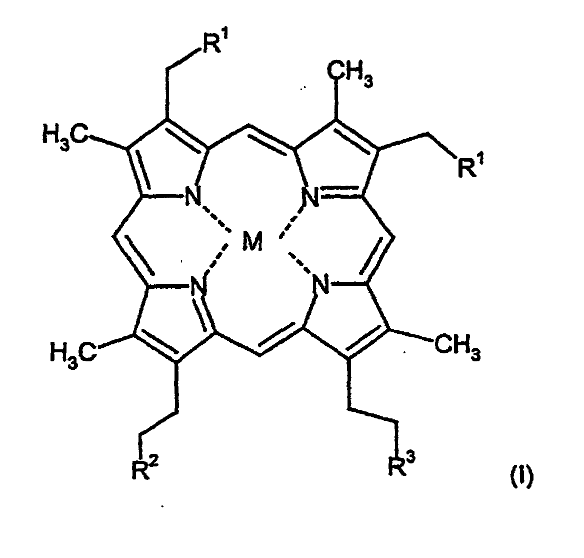 sowie mindestens einem Ion eines Elementes der Ordnungszahl 20-32, 37-39, 42-51 oder 57-83, worin
M für ein Fe³⁺, Mn³⁺, Cu²⁺, Co³⁺, VO²⁺, Cr³⁺ oder Ni²⁺- Ion,
R¹ für ein Wasserstoffatom, für einen geradkettigen C₁-C₆-Alkylrest, einen C₇-C₁₂-Aralkylrest oder für eine Gruppe OR' worin
R' ein Wasserstoffatom oder ein C₁-C₃-Alkylrest ist, steht,
R² für R³, eine Gruppe -CO-Z oder eine Gruppe -(NH)ₒ-(A)_{q}-NH-D steht, worin
Z eine Gruppe -OL ist, mit L in der Bedeutung eines anorganischen oder organischen Kations oder eines C₁-C₄-Alkylrestes ist,
A eine Phenylenoxy- oder eine durch ein oder mehrere Sauerstoffatome unterbrochene C₁-C₁₂-Alkylen- oder C₇-C₁₂ Aralkylengruppe bedeutet,
o und q unabhängig voneinander die Ziffern 0 oder 1 bedeuten und
D ein Wasserstoffatom oder eine Gruppe -CO-A-(COOL)ₒ-(H)ₘ bedeutet, mit m gleich 0 oder 1 und unter der Maßgabe, daß die Summe aus m und o gleich 1 ist,
R³ für eine Gruppe -(C=Q)(NR⁴)ₒ-(A)_{q}-(NR⁵)-K steht,
worin Q für ein Sauerstoffatom oder für zwei Wasserstoffatome steht,
R⁴ eine Gruppe -(A)_{q}-H bedeutet und
K einen Komplexbildner der allgemeinen Formel (IIa), (IIb), (IIc), (IId) ,(IIe) oder (IIf) bedeutet, wobei R⁵ für den Fall, daß K ein Komplexbildner der Formel (IIa) ist die gleiche Bedeutung wie R⁴ hat und R⁵ für den Fall, daß K ein Komplexbildner der Formel (IIb), (IIc), (IId), (IIe) oder (IIf) ist, die gleiche Bedeutung wie D hat,
mit der Maßgabe, daß eine direkte Sauerstoff-Stickstoff Bindung nicht zugelassen ist,
und K für einen Komplexbildner der allgemeinen Formel (IIa), (IIb), (IIc), (IId), (IIe) oder (IIf) 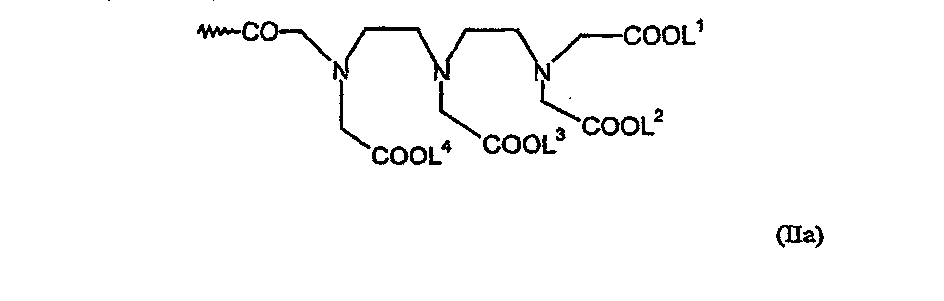 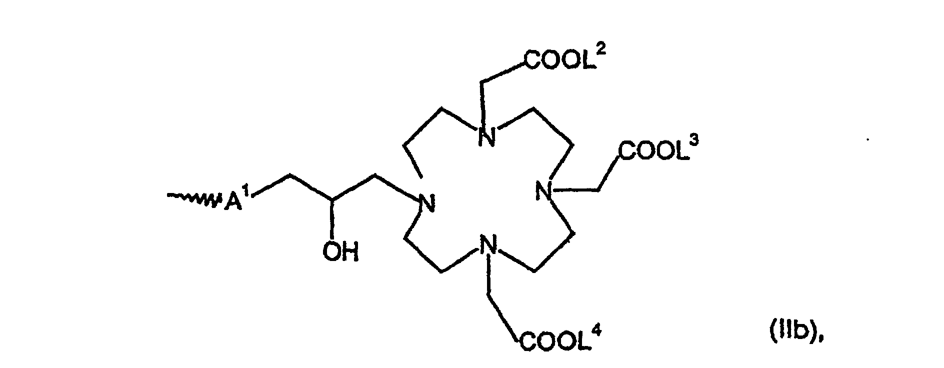 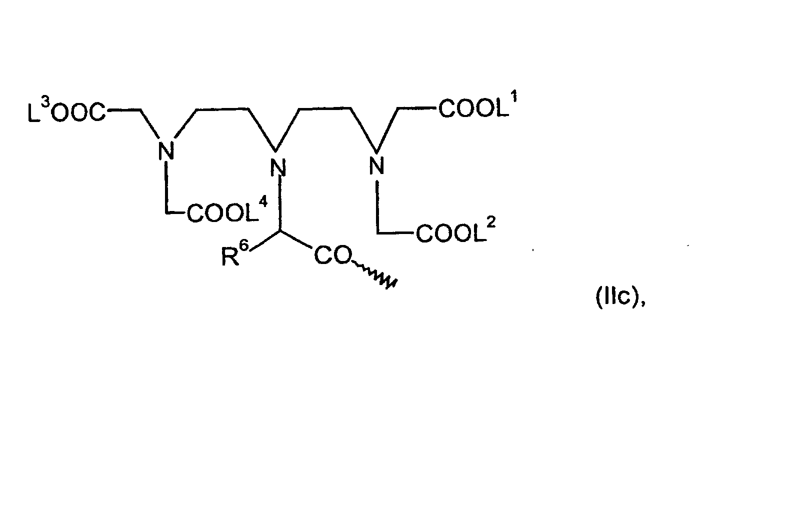 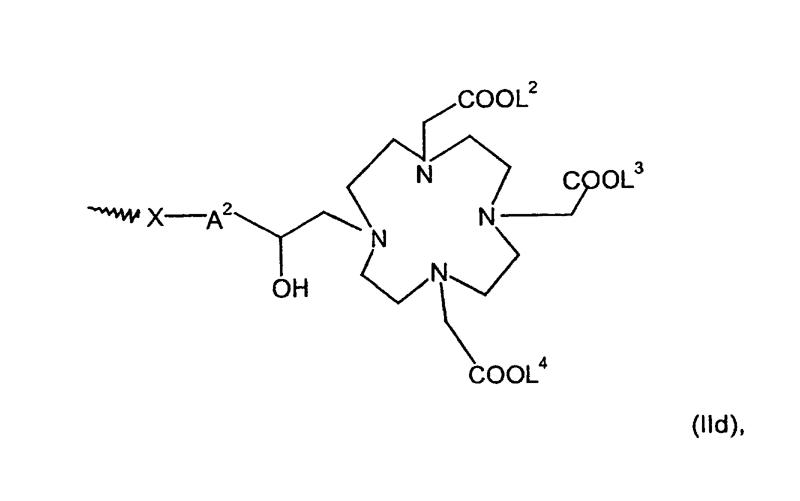 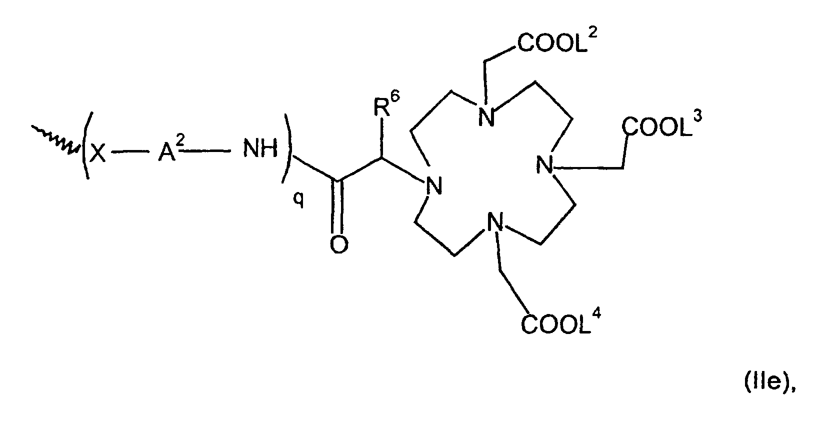 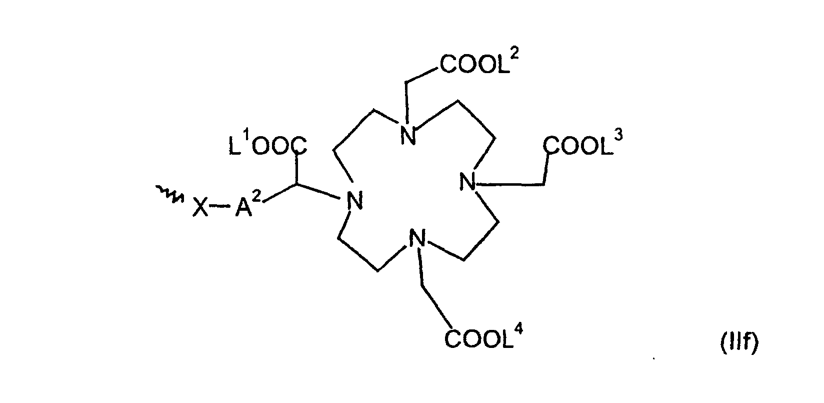
steht,
worin
q die oben angegebene Bedeutung hat,
A¹ die für A angegebene Bedeutung hat,
R⁶ für ein Wasserstoffatom, eine geradkettige oder verzweigte C₁- C₇-Alkylgruppe,
eine Phenyl- oder Benzylgruppe,
A² für eine Phenylen-, -CH₂-NHCO-CH₂-CH (CH₂COOH) -C₆H₄-β-, -C₆H₄-O-(CH₂)₀₋₅-β, -C₆H₄-(OCH₂CH₂)₀₋₁-N(CH₂COOH)-CH₂-β
oder eine gegebenenfalls durch ein oder mehrere Sauerstoffatome, 1 bis 3-NHCO-, 1 bis 3 -CONH-gruppen unterbrochene und/oder mit 1 bis 3(CH₂)ₒ₋₅COOH-Gruppen substituierte C₁-C₁₂-Alkylen- oder C₇-C₁₂-Alkylengruppe, wobei β für die Bindungsstelle an X steht,
X für eine -CO- oder NHCS-gruppe und
L¹, L², L³ und L⁴ unabhängig voneinander für einWasserstoffatom oder ein Metallionenäquivalent eines Elements der oben genannten Ordnungszahl steht, unter den Maßgaben, daß mindestens zwei dieser Substituenten für Metallionenäquivalente stehen, und daß zum Ausgleich gegebenenfalls vorhandener Ladungen im Metalloporphyrin weitere Anionen vorhanden sind und worin freie, nicht zur Komplexierung benötigte Carbonsäuregruppen auch als Salze mit physiologisch verträglich anorganischen und/oder organischen Kationen oder als Ester oder als Amide vorliegen können,
für die Herstellung von Mitteln für das Nekrose- und Infarkt-MR-Imaging.

2. Verwendung von Verbindungen gemäß Formel I von Anspruch 1 für die Herstellung von MRI-diagnostischen Mitteln für die Therapiekontrolle in der photodynamischen Therapie (PDT).

3. Verwendung von Porphyrin-Komplex-Verbindungen der allgemeinen Formel I gemäß Anspruch 1, **dadurch gekennzeichnet, daß** R² und R³ jeweils für eine -CONHNHK, -CONH(CH₂)₂NHK, -CONH(CH₂)₃NHK, -CONH(CH₂)₄NHK, -CONH(CH₂)₂O(CH₂)₂NHK-Gruppe stehen.

4. Porphyrin-Komplex-Verbindungen, bestehend aus einem Liganden der allgemeinen Formel I 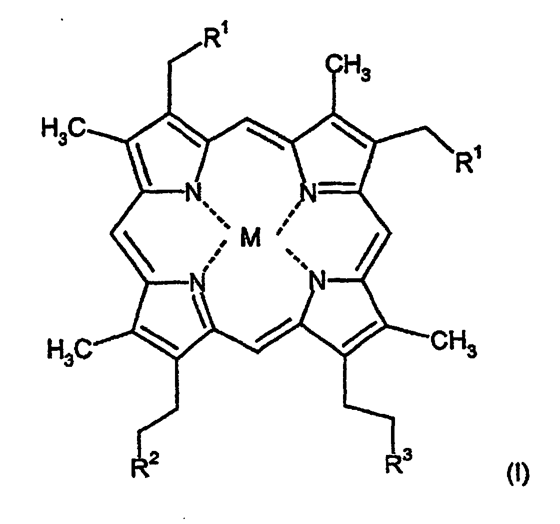 sowie mindestens einem Ion eines Elementes der Ordnungszahl 20-32, 37-39, 42-51 oder 57-83, worin
M für ein paramagnetisches Ion,
R¹ für ein Wasserstoffatom, für einen geradkettigen C₁-C₆-Alkylrest, einen C₇-C₁₂-Aralkylrest oder für eine Gruppe OR' worin
R' ein Wasserstoffatom oder ein C₁-C₃-Alkylrest ist, steht,
R² für R³, eine Gruppe -CO-Z oder eine Gruppe -(NH)ₒ-(A)_{q}-NH-D steht,
worin
Z eine Gruppe -OL ist, mit L in der Bedeutung eines anorganischen oder organischen Kations oder eines C₁-C₄-Alkylrestes ist,
A eine Phenylenoxy- oder eine durch ein oder mehrere Sauerstoffatome unterbrochene C₁-C₁₂-Alkylen- oder C₇-C₁₂ Aralkylengruppe bedeutet,
o und q unabhängig voneinander die Ziffern 0 oder 1 bedeuten und
D ein Wasserstoffatom oder eine Gruppe -CO-A-(COOL)ₒ-(H)ₘ bedeutet, mit m gleich 0 oder 1 und unter der Maßgabe, daß die Summe aus m und o gleich 1 ist,
R³ für eine Gruppe -(C=Q)(NR⁴)ₒ-(A)_{q}-(NR⁵)-K steht,
worin Q für ein Sauerstoffatom oder für zwei Wasserstoffatome steht,
R⁴ eine Gruppe -(A)_{q}-H bedeutet und
K einen Komplexbildner der allgemeinen Formel (IIc), (IId), (IIe) oder (IIf) bedeutet, wobei R⁵ die gleiche Bedeutung wie D hat, mit der Maßgabe, daß
eine direkte Sauerstoff-Stickstoff Bindung nicht zugelassen ist, und K für einen Komplexbildner der allgemeinen Formel (IIc), (IId), (IIe) oder (IIf) 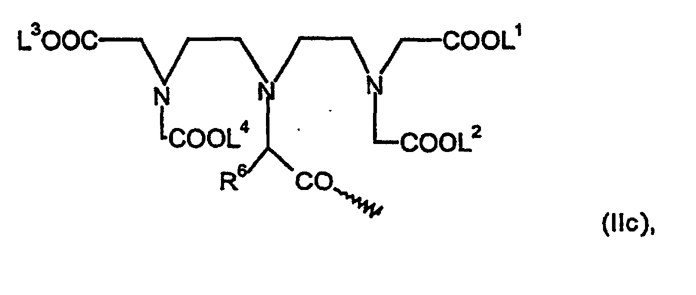 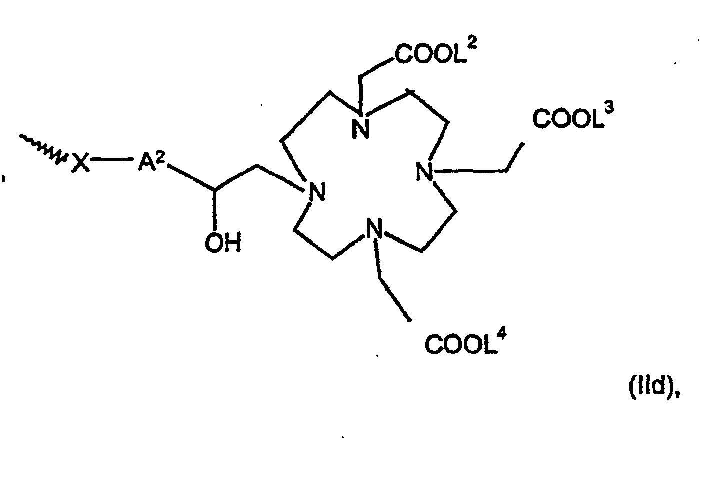 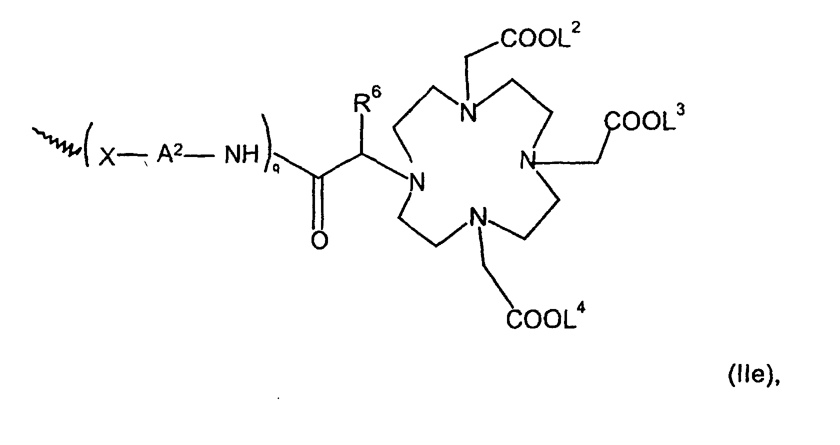 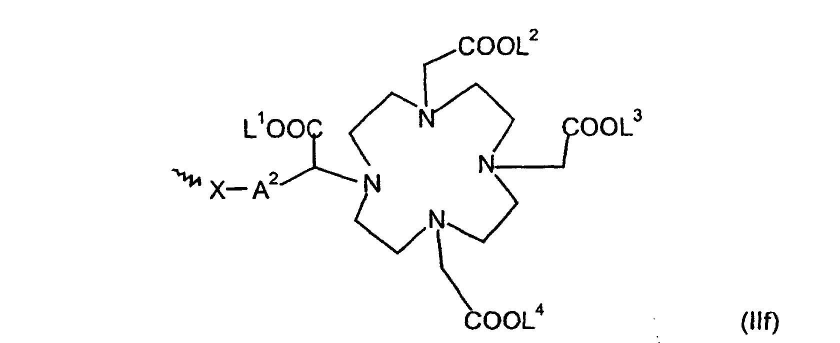 worin
q die oben angegebene Bedeutung hat,
A¹ die für A angegebene Bedeutung hat,
R⁶ für ein Wasserstoffatom, eine geradkettige oder verzweigte C₁- C₇-Alkylgruppe,
eine Phenyl- oder Benzylgruppe,
A² für eine Phenylen-, -CH₂-NHCO-CH₂-CH(CH₂COOH)-C₆H₄-β-, -C₆H₄-O-(CH₂)₀₋₅-β, -C₆H₄-(OCH₂CH₂)₀₋₁-N(CH₂COOH)-CH₂-β
oder eine gegebenenfalls durch ein oder mehrere Sauerstoffatome, 1 bis 3-NHCO-, 1 bis 3-CONH-gruppen unterbrochene und/oder mit 1 bis 3-(CH₂)₀₋₅COOH-Gruppen substituierte C₁-C₁₂-Alkylen- oder C₇-C₁₂-Alkylengruppe, wobei β für die Bindungsstelle an X steht,
X für eine -CO- oder NHCS-gruppe und
L¹, L², L³ und L⁴ unabhängig voneinander für ein Wasserstoffatom oder ein Metallionenäquivalent eines Elements der oben genannten Ordnungszahl steht, unter den Maßgaben, daß mindestens zwei dieser Substituenten für Metallionenäquivalente stehen, und daß zum Ausgleich gegebenenfalls vorhandener Ladungen im Metalloporphyrin weitere Anionen vorhanden sind und worin freie, nicht zur Komplexierung benötigte Carbonsäuregruppen auch als Salze mit physiologisch verträglich anorganischen und/oder organischen Kationen oder als Ester oder als Amide vorliegen können.

5. Komplexverbindungen gemäß Anspruch 4, **dadurch gekennzeichnet, daß** A² für eine -CH₂-, -(CH₂)₂-, -CH₂OC₆H₄-β, -CH₂OCH₂-, -C₆H₄-, -C₆H₄-OCH₂-β, -C₆H₄-OCH₂CH₂-N(CH₂COOH)-CH₂-β, CH₂-NHCO-CH₂-CH(CH₂COOH)-C₆H₄-β-gruppe steht, wobei β für die Bindungsstelle an X steht.

6. Komplex-Verbindungen gemäß Anspruch 4, **dadurch gekennzeichnet, daß** X für eine CO-Gruppe steht.

7. Komplex-Verbindungen gemäß Anspruch 4, **dadurch gekennzeichnet, daß** R⁶ für ein Wasserstoffatom oder eine Methylgruppe steht.

8. Porphyrin-Komplex-Verbindungen gemäß Formel I von Anspruch 1, nämlich {mu-[{16, 16'-[Chloromangan(III)-7, 12-diethyl-3, 8, 13, 17-tetramethylporphyrin-2, 18-diyl]-bis[3, 6, 9-tris(carboxymethyl)-11, 14-dioxo-3, 6, 9, 12, 13-pentaazahexadecanoato]}(8-)]}digadolinato(2-),-Dinatrium, {mu[{16, 16'-[Chloroeisen(III)-7, 12-diethyl-3, 8, 13, 17-tetramethylporphyrin-2, 18-diyl]-bis[3, 6, 9-tris(carboxymethyl)-11, 14-dioxo-3, 6, 9, 12, 13-pentaazahexadecanoato]}(8-)]}-digadolinato(2-),-Dinatrium, {mu[{16, 16'-[Kupfer(II)-7, 12-diethyl-3, 8, 13, 17-tetramethylporphyrin-2, 18-diyl]-bis[3, 6, 9-tris(carboxymethyl)-11, 14-dioxo-3, 6, 9, 12, 13-pentaazahexadecanoato]}(8-)]}-digadolinato(2-),-Dinatrium.

## Claims

1. Use of at least one porphyrin complex consisting of a ligand of the general formula I 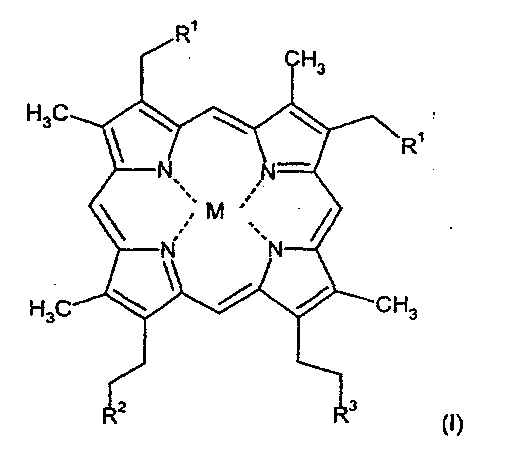 and of at least one ion of an element of atomic number 20-32, 37-39, 42-51 or 57-83, in which
M is a Fe³⁺, Mn³⁺, Cu²⁺, Co³⁺, VO²⁺, Cr³⁺ or Ni²⁺ ion,
R¹ is a hydrogen atom, a straight-chain C₁-C₆-alkyl radical, a C₇-C₁₂-aralkyl radical or a group OR' in which
R' is a hydrogen atom or a C₁-C₃-alkyl radical,
R² is R³, a group -CO-Z or a group -(NH)ₒ-(A)_{q}-NH-D, in which
Z is a group -OL, with L meaning an inorganic or organic cation or a C₁-C₄-alkyl radical,
A is a phenyleneoxy group or a C₁-C₁₂-alkylene or C₇-C₁₂-aralkylene group interrupted by one or more oxygen atoms,
o and q are, independently of one another, the numbers 0 or 1, and
D is a hydrogen atom or a group -CO-A-(COOL)ₒ-(H)ₘ, with m equal to 0 or 1 and with the proviso that the total of m and o equals 1,
R³ is a group -(C=Q)(NR⁴)ₒ-(A)_{q}-(NR⁵)-K, in which Q is an oxygen atom or two hydrogen atoms,
R⁴ is a group -(A)_{q}-H and
K is a complexing agent of the general formula (IIa), (IIb), (IIc), (IId), (IIe) or (IIf), where R⁵ has the same meaning as R⁴ in the case where K is a complexing agent of the formula (IIa), and R⁵ has the same meaning as D in the case where K is a complexing agent of the formula (IIb), (IIc), (IId), (IIe) or (IIf),
with the proviso that a direct oxygen-nitrogen linkage is not allowed, and K is a complexing agent of the general formula (IIa), (IIb), (IIc), (IId), (IIe) or (IIf) 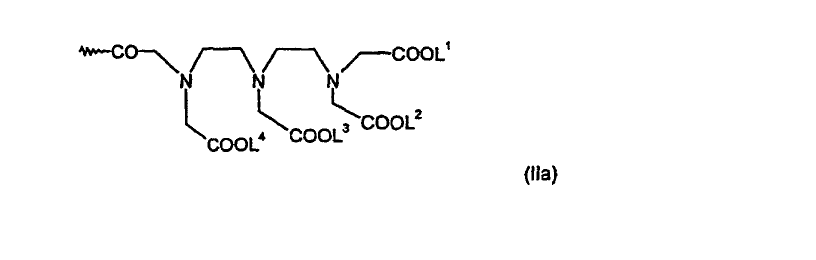 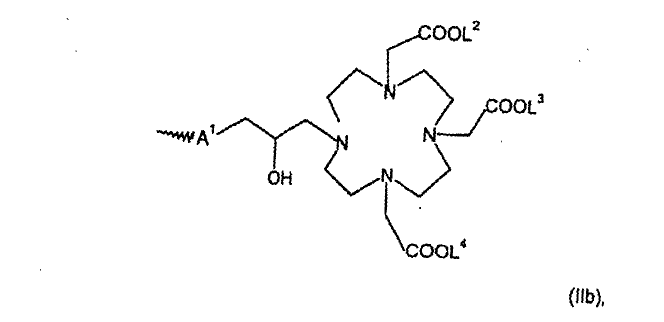 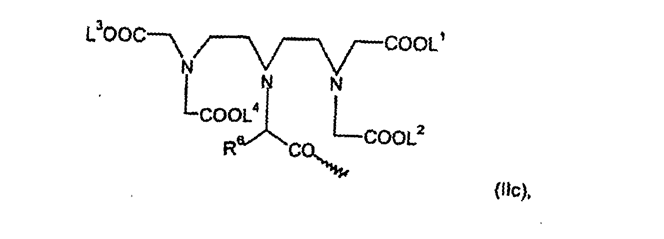 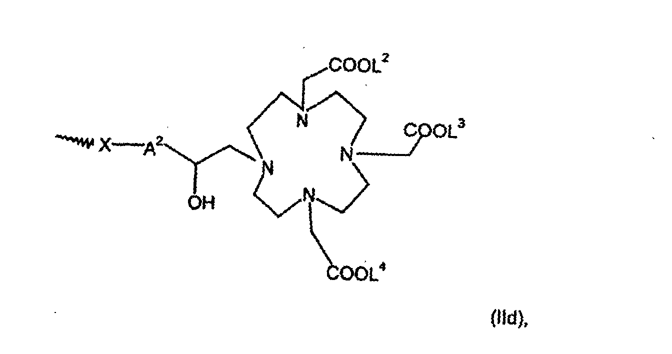 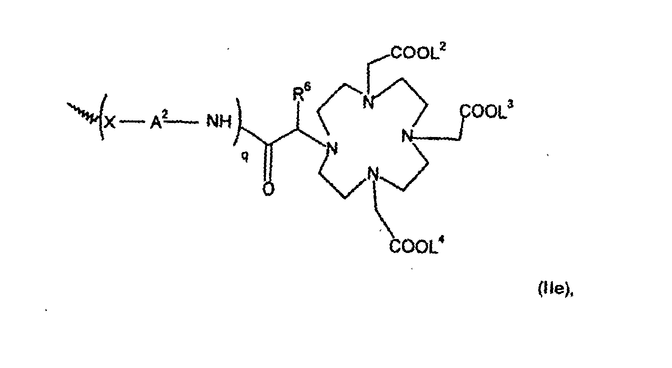 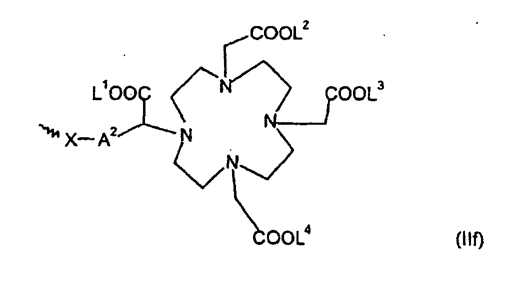 in which
q has the meaning stated above,
A¹ has the meaning stated for A,
R⁶ is a hydrogen atom, a straight-chain or branched C₁-C₇ alkyl group,
a phenyl or benzyl group,
A² is a phenylene, -CH₂-NHCO-CH₂-CH(CH₂COOH)-C₆H₄-β-, -C₆H₄-O-(CH₂)₀₋₅-β, -C₆H₄-(OCH₂CH₂)₀₋₁-N(CH₂COOH)-CH₂-β group or a C₁-C₁₂-alkylene or C₇-C₁₂-alkylene group which is optionally interrupted by one or more oxygen atoms, 1 to 3-NHCO-, 1 to 3-CONH- groups and/or substituted by 1 to 3-(CH₂)₀₋₅COOH groups, where β is the linkage site to X,
X is a -CO- or NHCS- group and
L¹, L², L³ and L⁴ are, independently of one another, a hydrogen atom or a metal ion equivalent of an element of the abovementioned atomic number, with the provisos that at least two of these substituents are metal ion equivalents, and that further anions are present to balance charges present where appropriate in the metalloporphyrin, and in which free carboxylic acid groups not required for complexation may also be in the form of salts with physiologically tolerated inorganic and/or organic cations or of esters or of amides, for producing compositions for MR imaging of necroses and infarctions.

2. Use of compounds according to formula I of Claim 1 for producing MRI diagnostic compositions for monitoring therapy in photodynamic therapy (PDT).

3. Use of porphyrin complex compounds of the general formula I according to Claim 1, **characterized in that** R² and R³ are each a -CONHNHK, -CONH(CH₂)₂NHK, -CONH(CH₂)₃NHK, -CONH(CH₂)₄NHK, -CONH(CH₂)₂O(CH₂)₂NHK group.

4. Porphyrin complex compounds consisting of a ligand of the general formula I 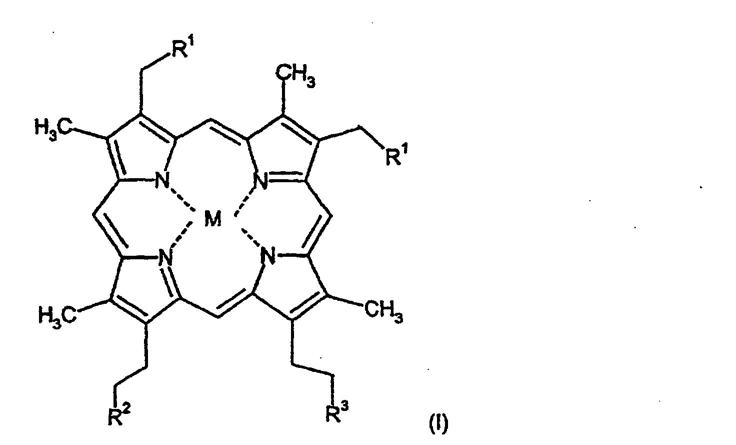 and of at least one ion of an element of atomic number 20-32, 37-39, 42-51 or 57-83, in which
M is a paramagnetic ion,
R¹ is a hydrogen atom, a straight-chain C₁-C₆-alkyl radical, a C₇-C₁₂-aralkyl radical or a group OR' in which
R' is a hydrogen atom or a C₁-C₃-alkyl radical,
R² is R³, a group -CO-Z or a group -(NH)ₒ-(A)_{q}-NH-D,
in which
Z is a group -OL, with L meaning an inorganic or organic cation or a C₁-C₄-alkyl radical,
A is a phenyleneoxy group or a C₁-C₁₂-alkylene or C₇-C₁₂-aralkylene group interrupted by one or more oxygen atoms,
o and q are, independently of one another, the numbers 0 or 1, and
D is a hydrogen atom or a group -CO-A-(COOL)ₒ-(H)ₘ, with m equal to 0 or 1 and with the proviso that the total of m and o equals 1,
R³ is a group -(C=Q)(NR⁴)ₒ-(A)_{q}-(NR⁵)-K, in which Q is an oxygen atom or two hydrogen atoms,
R⁴ is a group -(A)_{q}-H and
K is a complexing agent of the general formula (IIc), (IId), (IIe) or (IIf), where R⁵ has the same meaning as D, with the proviso that a direct oxygen-nitrogen linkage is not allowed, and K is a complexing agent of the general formula (IIc), (IId), (IIe) or (IIf) 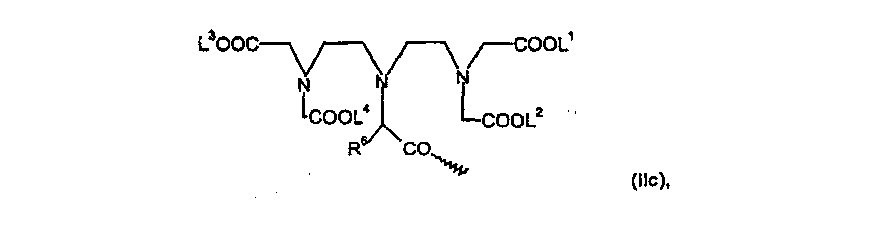 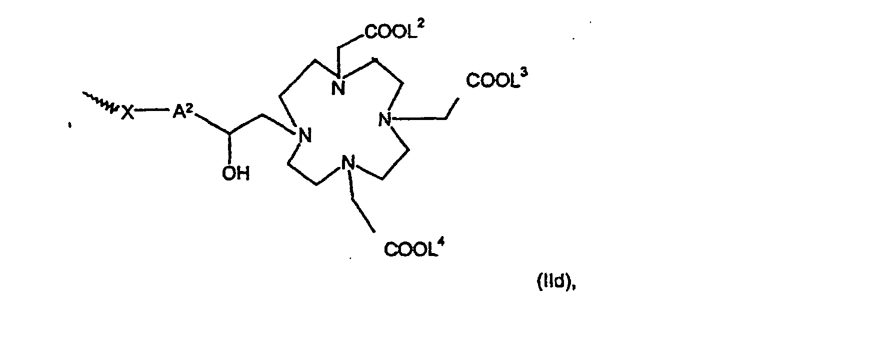 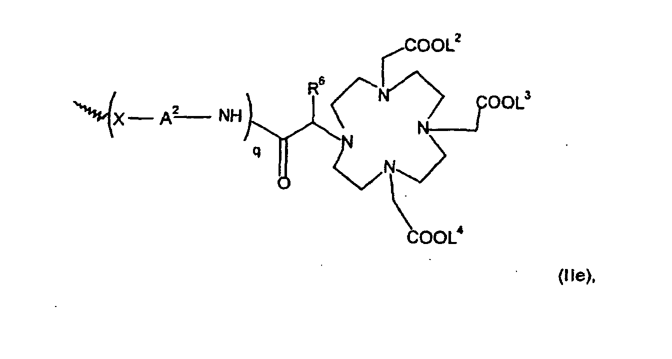 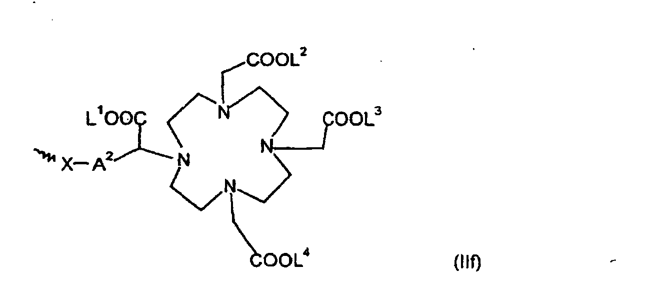 in which
q has the meaning stated above,
A¹ has the meaning stated for A,
R⁶ is a hydrogen atom, a straight-chain or branched C₁-C₇ alkyl group,
a phenyl or benzyl group,
A² is a phenylene, -CH₂-NHCO-CH₂-CH(CH₂COOH)-C₆H₄-β-, -C₆H₄-O-(CH₂)₀₋₅-β, -C₆H₄-(OCH₂CH₂)₀₋₁-N(CH₂COOH)-CH₂-β group or a C₁-C₁₂-alkylene or C₇-C₁₂-alkylene group which is optionally interrupted by one or more oxygen atoms, 1 to 3-NHCO-, 1 to 3-CONH- groups and/or substituted by 1 to 3-(CH₂)₀₋₅COOH groups, where β is the linkage site to X,
X is a -CO- or NHCS- group and
L¹, L², L³ and L⁴ are, independently of one another, a hydrogen atom or a metal ion equivalent of an element of the abovementioned atomic number, with the provisos that at least two of these substituents are metal ion equivalents, and that further anions are present to balance charges present where appropriate in the metalloporphyrin, and in which free carboxylic acid groups not required for complexation may also be in the form of salts with physiologically tolerated inorganic and/or organic cations or of esters or of amides.

5. Complex compounds according to Claim 4, **characterized in that** A² is a -CH₂-, -(CH₂)₂-, -CH₂OC₆H₄-β, -CH₂OCH₂-, -C₆H₄-, -C₆H₄-OCH₂-β, -C₆H₄-OCH₂CH₂-N(CH₂COOH)-CH₂-β, CH₂-NHCO-CH₂-CH(CH₂COOH)-C₆H₄-β- group, where β is the linkage site to X.

6. Complex compounds according to Claim 4, **characterized in that** X is a CO group.

7. Complex compounds according to Claim 4, **characterized in that** R⁶ is a hydrogen atom or a methyl group.

8. Porphyrin complex compounds according to formula 1 of Claim 1, namely {mu-[{16,16,'-[chloro-manganese(III)-7,12-diethyl-3,8,13,17-tetra-methylporphyrin-2,18-diyl]-bis[3,6,9-tris(carboxymethyl)-11,14-dioxo-3,6,9,12,13-pentaazahexadecanoato]}(8-)]}-digadolinato(2-) disodium, {mu[{16,16'-[chloroiron(III)-7,12-diethyl-3,8,13,17-tetramethylporphyrin-2,18-diyl]-bis-[3,6,9-tris(carboxymethyl)-11,14-dioxo-3,6,9,12,13-pentaazahexadecanoato]}(8-)]}-digadolinato(2-) disodium, {mu[{16,16'-[copper(II)-7,12-diethyl-3,8,13,17-tetramethylporphyrin-2, 18-diyl]-bis[3,6,9-tris(carboxymethyl)-11,14-dioxo-3,6,9,12,13-pentaazahexadecanoato)}(8-)]}-digadolinato(2-) disodium.

## Revendications

1. Utilisation d'au moins un complexe de porphyrine constitué d'un ligand de formule générale I 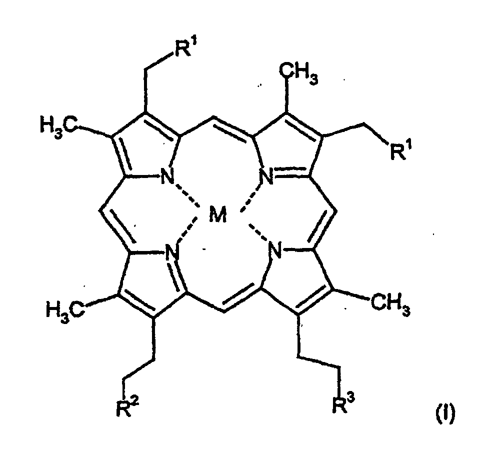 et d'au moins un ion d'un élément de numéro atomique 20 - 32, 37 - 39, 42 - 51 ou 57 - 83, dans laquelle
M représente un ion Fe³⁺, Mn³⁺, Cu²⁺, Co³⁺, VO²⁺, Cr³⁺ ou Ni²⁺,
R¹ représente un atome d'hydrogène, un radical alkyle en C₁-C₆ linéaire, un radical aralkyle en C₇-C₁₂ ou un groupe OR' dans lequel
R' représente un atome d'hydrogène ou un radical alkyle en C₁-C₃,
R² représente R³, un groupe -CO-Z ou un groupe -(NH)ₒ-(A)_{q}-NH-D, dans lequel
Z représente un groupe -OL, dans lequel L représente un cation inorganique ou organique ou un radical alkyle en C₁-C₄,
A représente un groupe phénylène-oxy ou un groupe alkylène en C₁-C₁₂ ou aralkylène en C₇-C₁₂ interrompu par un ou plusieurs atomes d'oxygène,
o et q représentent, indépendamment l'un de l'autre, les chiffres 0 ou 1 et
D représente un atome d'hydrogène ou un groupe -CO-A-(COOL)ₒ-(H)ₘ, dans lequel m vaut 0 ou 1 et à condition que la somme de m et o vaille 1,
R³ représente un groupe -(C=Q)(NR⁴)ₒ-(A)_{q}-(NR⁵)-K,
dans lequel Q représente un atome d'oxygène ou deux atomes d'hydrogène,
R⁴ représente un groupe -(A)_{q}-H et
K représente un agent complexant de formules générales (IIa), (IIb), (IIc), (IId), (IIe) ou (IIf), où R⁵ présente la même signification que R⁴ dans le cas où K représente un agent complexant de formule (IIa) et R⁵ présente la même signification que D dans le cas où K représente un agent complexant de formule (IIb), (IIc), (IId), (IIe) ou (IIf),
à condition qu'une liaison directe oxygène-azote ne soit pas permise,
et K représente un agent complexant de formules générales (IIa), (IIb), (IIc), (IId), (IIe) ou (IIf) 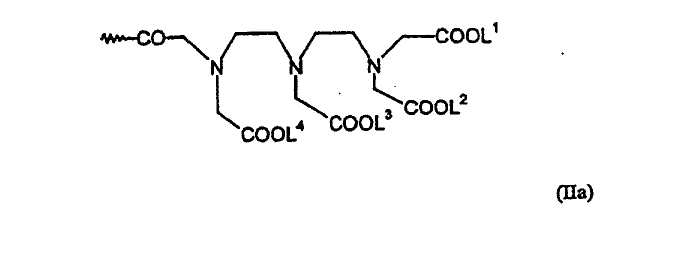 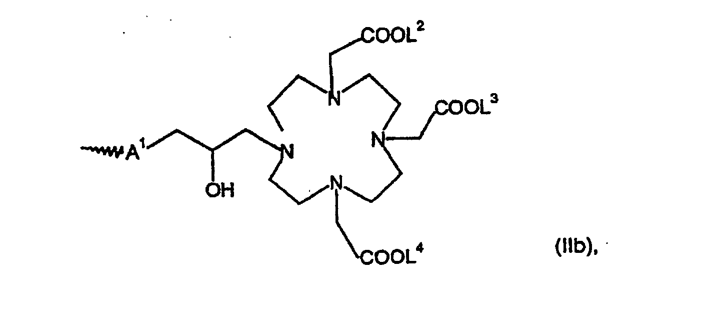 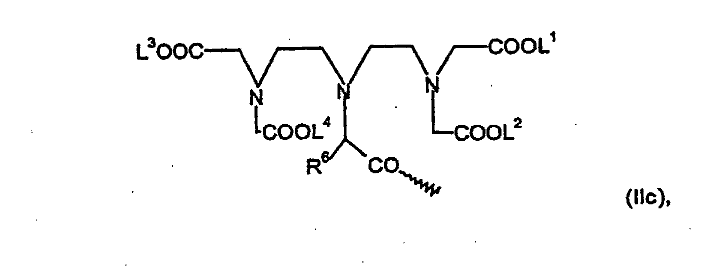 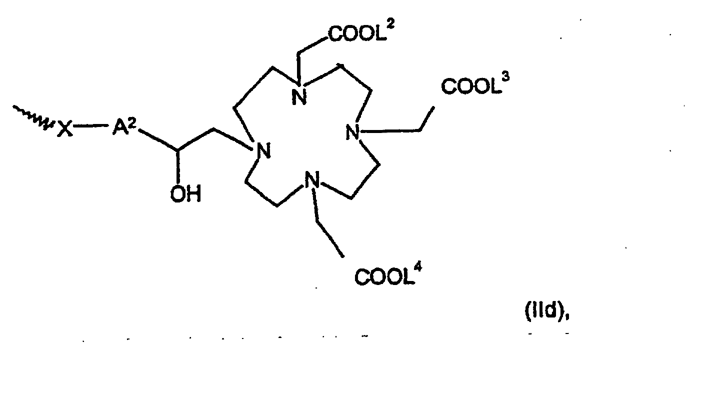 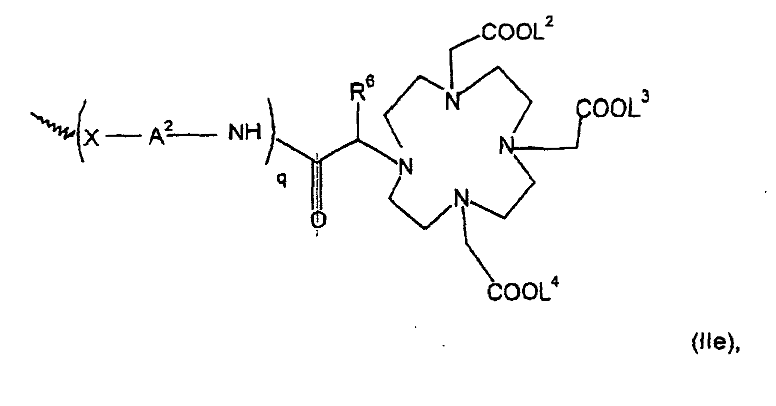 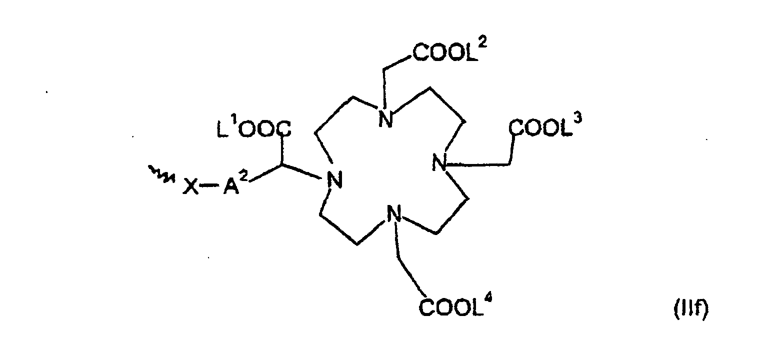
dans lesquelles
q présente la signification indiquée ci-dessus,
A¹ présente la signification indiquée pour A,
R⁶ représente un atome d'hydrogène, un groupe alkyle en C₁-C₇ linéaire ou ramifié, un groupe phényle ou benzyle,
A² représente un phénylène, un groupe -CH₂-NHCO-CH₂-CH(CH₂COOH)-C₆H₄-β, -C₆H₄-O-(CH₂)₀₋₅-β, -C₆H₄-(OCH₂CH₂)₀₋₁-N(CH₂COOH)-CH₂-β,
ou un groupe alkylène en C₁-C₁₂ ou alkylène en C₇-C₁₂ éventuellement interrompu par un ou plusieurs atomes d'oxygène, de 1 à 3 groupes NHCO-, de 1 à 3 groupes -CONH- et/ou substitué par de 1 à 3 groupes -C(CH₂)₀₋₅COOH, β représentant le site de liaison à X,
X représente un groupe -CO- ou NHCS- et
L¹, L², L³ et L⁴ représentent, indépendamment les uns des autres, un atome d'hydrogène ou un équivalent d'ion métallique d'un élément du numéro atomique susmentionné, à condition qu'au moins deux de ces substituants soient des équivalents d'ions métalliques, et que d'autres anions soient présents pour équilibrer les charges éventuellement présentes dans la métalloporphyrine, et où des groupes acide carboxylique libres non nécessaires pour la complexation peuvent également se trouver sous la forme de sels avec des cations inorganiques et/ou organiques physiologiquement compatibles ou sous forme d'esters ou bien d'amides, pour la préparation de compositions destinées à l'imagerie RM de nécroses et d'infarctus.

2. Utilisation de composés de formule I selon la revendication 1 pour la préparation de compositions diagnostiques par MRI pour le contrôle thérapeutique en thérapie photodynamique (TPD).

3. Utilisation de composés complexes de porphyrine de formule générale I selon la revendication 1, **caractérisée en ce que** R² et R³ représentent chacun un groupe -CONHNHK, -CONH (CH₂)₂NHK, -CONH (CH₂)₃NHK, -CONH(CH₂)₄NHK, -CONH(CH₂)₂O(CH₂)₂NHK.

4. Composés complexes de porphyrine constitués d'un ligand de formule générale I 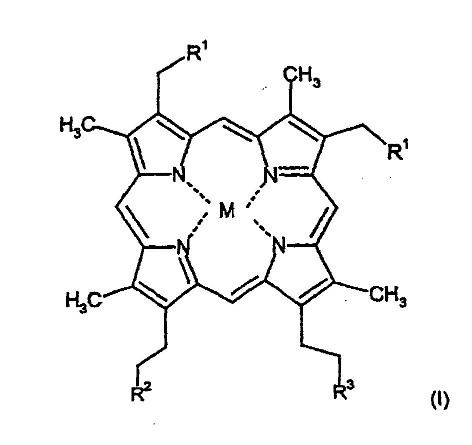 et d'au moins un ion d'un élément de numéro atomique 20 - 32, 37 - 39, 42 - 51 ou 57 - 83, dans laquelle
M représente un ion paramagnétique,
R¹ représente un atome d'hydrogène, un radical alkyle en C₁-C₆ linéaire, un radical aralkyle en C₇-C₁₂ ou un groupe OR' dans lequel
R' représente un atome d'hydrogène ou un radical alkyle en C₁-C₃,
R² représente R³, un groupe -CO-Z ou un groupe -(NH)ₒ-(A)_{q}-NH-D, dans lequel
Z représente un groupe -OL, dans lequel L représente un cation inorganique ou organique ou un radical alkyle en C₁-C₄,
A représente un groupe phénylène-oxy ou un groupe alkylène en C₁-C₁₂ ou aralkylène en C₇-C₁₂ interrompu- par un ou plusieurs atomes d'oxygène,
o et q représentent, indépendamment l'un de l'autre, les chiffres 0 ou 1 et
D représente un atome d'hydrogène ou un groupe -CO-A-(COOL)ₒ-(H)ₘ, dans lequel m vaut 0 ou 1 et à condition que la somme de m et o vaille 1,
R³ représente un groupe -(C=Q) (NR⁴)ₒ-(A)_{q}-(NR⁵)-K,
dans lequel Q représente un atome d'oxygène ou deux atomes d'hydrogène,
R⁴ représente un groupe -(A)_{q}-H et
K représente un agent complexant de formules générales (IIc), (IId), (IIe) ou (IIf), dans lesquelles R⁵ présente la même signification que D, à condition que
une liaison oxygène-azote directe ne soit pas permise,
et K présente un agent complexant de formules générales (IIc), (IId), (IIe) ou (IIf) 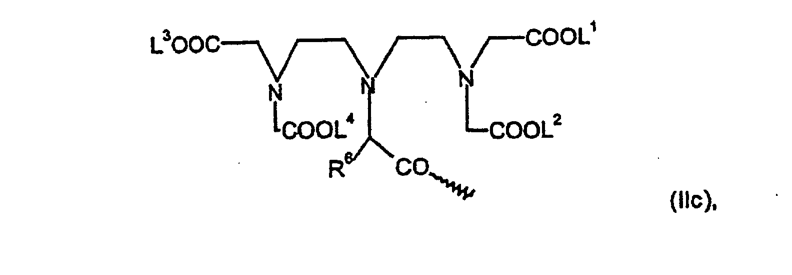 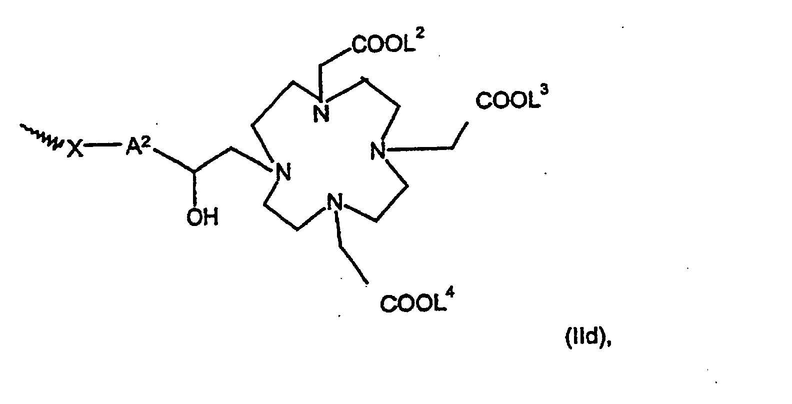 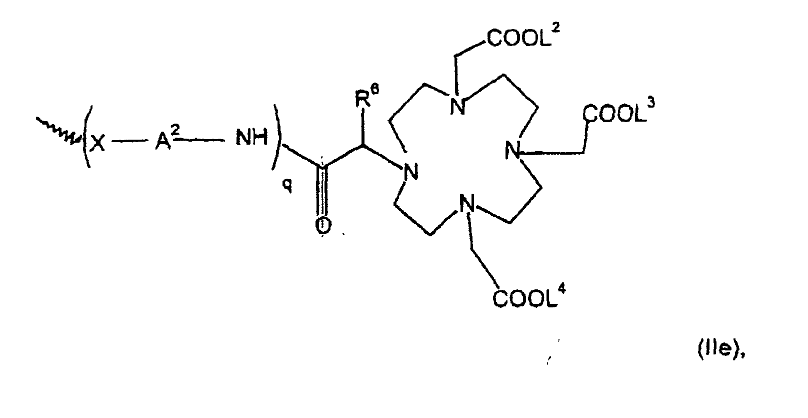 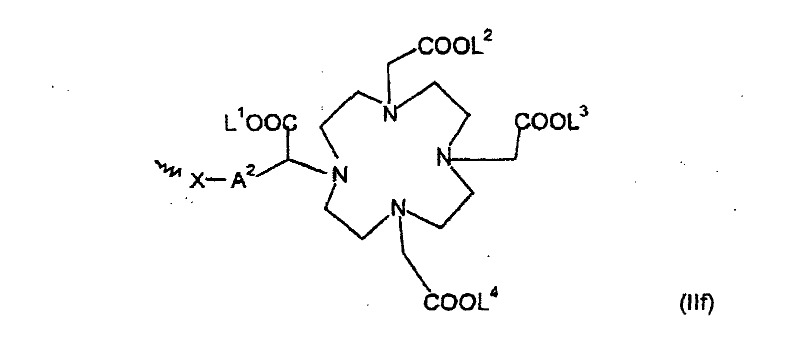 dans lesquelles
q présente la signification indiquée ci-dessus,
A¹ présente la signification indiquée pour A,
R⁶ représente un atome d'hydrogène, un groupe alkyle en C₁-C₇ linéaire ou ramifié, un groupe phényle ou benzyle,
A² représente un phénylène, un groupe -CH₂-NHCO-CH₂-CH(CH₂COOH)-C₆H₄-β, -C₆H₄-O-(CH₂)₀₋₅-β, -C₆H₄-(OCH₂CH₂)₀₋₁-N(CH₂COOH)-CH₂-β,
ou un groupe alkylène en C₁-C₁₂ ou alkylène en C₇-C₁₂ éventuellement interrompu par un ou plusieurs atomes d'oxygène, de 1 à 3 groupes NHCO-, de 1 à 3 groupes -CONH- et/ou substitué par de 1 à 3 groupes -C(CH₂)₀₋₅COOH, β représentant le site de liaison à X,
X représente un groupe -CO- ou NHCS- et
L¹, L², L³ et L⁴ représentent, indépendamment les uns des autres, un atome d'hydrogène ou un équivalent d'ion métallique d'un élément du numéro atomique susmentionné, à condition qu'au moins deux de ces substituants soient des équivalents d'ions métalliques, et que d'autres anions soient présents pour équilibrer les charges éventuellement présentes dans la métalloporphyrine, et où des groupes acide carboxylique libres non nécessaires pour la complexation peuvent également se trouver sous la forme de sels avec des cations inorganiques et/ou organiques physiologiquement compatibles ou sous forme d'esters ou bien d'amides.

5. Composés complexes selon la revendication 4,
**caractérisés en ce que** A² représente un groupe -CH₂-, -(CH₂)₂-, -CH₂OC₆H₄-β, -CH₂OCH₂-, -C₆H₄-, -C₆H₄-OCH₂-β, -C₆H₄-OCH₂CH₂-N(CH₂COOH)-CH₂-β, CH₂-NHCO-CH₂-CH(CH₂COOH)-C₆H₄-β, β représentant le site de liaison à X.

6. Composés complexes selon la revendication 4, **caractérisés en ce que** X représente un groupe CO.

7. Composés complexes selon la revendication 4, **caractérisés en ce que** R⁶ représente un atome d'hydrogène ou un groupe méthyle.

8. Composés complexes de porphyrine de formule I selon la revendication 1, à savoir le {mu-[{16,16'-[chloromanganèse(III)-7,12-diéthyl-3,8,13,17-tétraméthylporphyrine-2,18-diyl]-bis[3,6,9-tris(carboxyméthyl)-11,14-dioxo-3,6,9,12,13-pentaazahexadécanoato]}(8-)]}digadolinato(2-) disodique, le {mu-[{16,16'-[chlorofer(III)-7,12-diéthyl-3,8,13,17-tétraméthylporphyrine-2,18-diyl]-bis[3,6,9-tris(carboxyméthyl)-11,14-dioxo-3,6,9,12,13-pentaazahexadécanoato]}(8-)]}digadolinato(2-) disodique, le {mu-[{16,16'-[cuivre(II)-7,12-diéthyl-3,8,13,17-tétraméthylporphyrine-2,18-diyl]-bis[3,6,9-tris(carboxyméthyl)-11,14-dioxo-3,6,9,12,13-pentaazahexadécanoato]}(8-)]}digadolinato(2-) disodique.
